# EUROPEAN PATENT SPECIFICATION

(11) **EP 1 392 728 B1**
(45) Date of publication and mention of the grant of the patent: **08.08.2007**
(21) Application number: 02729715.9
(22) Date of filing: 29.05.2002
(51) Int. Cl.: C07K 14/47, A61K 39/00, A61P 25/28

(54) **VACCINE FOR THE PREVENTION AND TREATMENT OF ALZHEIMER'S AND AMYLOID RELATED DISEASES**
IMPFSTOFF ZUR VERHÜTUNG UND BEHANLDUNG VON ALZHEIMER-DEMENZ UND VON AMYLOID-BEZOGENEN KRANKHEITEN
VACCIN DESTINE A LA PREVENTION ET AU TRAITEMENT DE LA MALADIE D'ALZHEIMER ET D'AUTRES MALADIES ASSOCIEES AU SUBSTANCES AMYLOIDES

(30) Priority: 29.05.2001 US 867847
(43) Date of publication of application: 03.03.2004
(73) Proprietor: Neurochem (International) Limited, 1015 Lausanne (Ecublens) (CH)
(72) Inventor: GERVAIS, Francine, Ile Bizard, Quebec H9C 2X5 (CA); HEBERT, Lise, Montreal, Quebec H1G 5X2 (CA); CHALIFOUR, Robert, J., Ile Bizard, Québec H9C 1T4 (CA); KONG, Xianqi, Dollard-des-Ormeaux, Québec H9B 3J7 (CA)
(74) Representative: Killin, Stephen James
(86) International application number: PCT/CA2002/000763
(87) International publication number: WO 2002/096937

(56) References cited:
- WO-A-00/68263
- WO-A-00/72880
- WO-A-01/39796
- CRIBBS DH ET AL.: "All-D-Enantiomers of beta-Amyloid Exhibit Similar Biological Properties to All-L-beta-Amyloids" THE JOURNAL OF BIOLOGICAL CHEMISTRY, vol. 272, no. 11, 14 March 1997 (1997-03-14), pages 7431-7436, XP002230811
- BENKIRANE N ET AL.: "Antigenicity and Immunogenicity of Modified Synthetic Peptides Containing D-Amino Acid Residues" THE JOURNAL OF BIOLOGICAL CHEMISTRY, vol. 268, no. 35, 15 December 1993 (1993-12-15), pages 26279-26285, XP002164690

### Annex

### SEQUENCE LISTING

<110> Neurochem Inc.
   Gervais, Francine
   Hebert, Lise
   Chalifour, Robert J.
   Kong, Xianqi
<120> Vaccine for the Prevention and Treatment of Alzheimer's and Amyloid Related Diseases
<130> 14228-6PCT-1
<140> PCT/CA02/00763
   <141> 2002-05-29
<150> 09/867,847
   <151> 2001-05-29
<160> 70
<170> FastSEQ for Windows Version 4.0
<210> 1
   <211> 42
   <212> PRT
   <213> Artificial Sequence
<220>
   <223> Synthetic, All D peptides or peptidomimetics
<400> 1
<210> 2
   <211> 40
   <212> PRT
   <213> Artificial Sequence
<220>
   <223> Synthetic, All D peptides or peptidomimetics
<400> 2
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35 40
<210> 3
   <211> 35
   <212> PRT
   <213> Artificial Sequence
<220>
   <223> Synthetic, All D peptides or peptidomimetics
<400> 3
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met
   35
<210> 4
   <211> 28
   <212> PRT
   <213> Artificial Sequence
<220>
   <223> Synthetic, All D peptides or peptidomimetics
<400> 4
   Asp Ala Glu Phe Arg His Asp Ser Gly Tyr Glu Val His His Gln Lys
   1 5 10 15
   Leu Val Phe Phe Ala Glu Asp Val Gly Ser Asn Lys
   20 25
<210> 5
   <211> 7
   <212> PRT
   <213> Artificial Sequence
<220>
   <223> Synthetic, All D peptides or peptidomimetics
<400> 5
   Asp Ala Glu Phe Arg His Asp
   1 5
<210> 6
   <211> 7
   <212> PRT
   <213> Artificial Sequence
<220>
   <223> Synthetic, All D peptides or peptidomimetics
<400> 6
   Tyr Glu Val His His Gln Lys
   1 5
<210> 7
   <211> 6
   <212> PRT
   <213> Artificial Sequence
<220>
   <223> Synthetic, All D peptides or peptidomimetics
<400> 7
   Lys Leu Val Phe Phe Ala
   1 5
<210> 8
   <211> 12
   <212> PRT
   <213> Artificial Sequence
<220>
   <223> Synthetic, All D peptides or peptidomimetics
<400> 8
   Tyr Glu Val His His Gln Lys Leu Val Phe Phe Ala
   1 5 10
<210> 9
   <211> 9
   <212> PRT
   <213> Artificial Sequence
<220>
   <223> Synthetic, All D peptides or peptidomimetics
<400> 9
   His His Gln Lys Leu Val Phe Phe Ala
   1 5
<210> 10
   <211> 7
   <212> PRT
   <213> Artificial Sequence
<220>
   <223> Synthetic, All D peptides or peptidomimetics
<400> 10
   Val Gly Gly Val Val Ile Ala
   1 5
<210> 11
   <211> 6
   <212> PRT
   <213> Artificial Sequence
<220>
   <223> Synthetic, All D peptides or peptidomimetics
<400> 11
   Lys Ile Val Phe Phe Ala
   1 5
<210> 12
   <211> 7
   <212> PRT
   <213> Artificial Sequence
<220>
   <223> Synthetic, All D peptides or peptidomimetics
<400> 12
   Lys Lys Leu Val Phe Phe Ala
   1 5
<210> 13
   <211> 6
   <212> PRT
   <213> Artificial Sequence
<220>
   <223> Synthetic, All D peptides or peptidomimetics
<400> 13
   Lys Phe Val Phe Phe Ala
   1 5
<210> 14
   <211> 6
   <212> PRT
   <213> Artificial Sequence
<220>
   <223> Synthetic, All D peptides or peptidomimetics
<400> 14
   Ala Phe Phe Val Leu Lys
   1 5
<210> 15
   <211> 4
   <212> PRT
   <213> Artificial Sequence
<220>
   <223> Synthetic, All D peptides or peptidomimetics
<400> 15
   Lys Leu Val Phe
   1
<210> 16
   <211> 6
   <212> PRT
   <213> Artificial Sequence
<220>
   <223> Synthetic, All D peptides or peptidomimetics
<400> 16
   Lys Ala Val Phe Phe Ala
   1 5
<210> 17
   <211> 5
   <212> PRT
   <213> Artificial Sequence
<220>
   <223> Synthetic, All D peptides or peptidomimetics
<400> 17
   Lys Leu Val Phe Phe
   1 5
<210> 18
   <211> 6
   <212> PRT
   <213> Artificial Sequence
<220>
   <223> Synthetic, All D peptides or peptidomimetics
<400> 18
   Lys Val Val Phe Phe Ala
   1 5
<210> 19
   <211> 6
   <212> PRT
   <213> Artificial Sequence
<220>
   <223> Synthetic, All D peptides or peptidomimetics
<221> MOD_RES
   <222> 6
   <223> Amidation
<400> 19
   Lys Ile Val Phe Phe Ala
   1 5
<210> 20
   <211> 6
   <212> PRT
   <213> Artificial Sequence
<220>
   <223> Synthetic, All D peptides or peptidomimetics
<221> MOD_RES
   <222> 6
   <223> Amidation
<400> 20
   Lys Leu Val Phe Phe Ala
   1 5
<210> 21
   <211> 6
   <212> PRT
   <213> Artificial Sequence
<220>
   <223> Synthetic, All D peptides or peptidomimetics
<221> MOD_RES
   <222> 6
   <223> Amidation
<400> 21
   Lys Phe Val Phe Phe Ala
   1 5
<210> 22
   <211> 6
   <212> PRT
   <213> Artificial Sequence
<220>
   <223> Synthetic, All D peptides or peptidomimetics
<221> MOD_RES
   <222> 6
   <223> Amidation
<400> 22
   Ala Phe Phe Val Leu Lys
   1 5
<210> 23
   <211> 4
   <212> PRT
   <213> Artificial Sequence
<220>
   <223> Synthetic, All D peptides or peptidomimetics
<221> MOD_RES
   <222> 4
   <223> Amidation
<400> 23
   Lys Leu Val Phe
   1
<210> 24
   <211> 6
   <212> PRT
   <213> Artificial Sequence
<220>
   <223> Synthetic, All D peptides or peptidomimetics
<221> MOD_RES
   <222> 6
   <223> Amidation
<400> 24
   Lys Ala Val Phe Phe Ala
   1 5
<210> 25
   <211> 5
   <212> PRT
   <213> Artificial Sequence
<220>
   <223> Synthetic, All D peptides or peptidomimetics
<221> MOD_RES
   <222> 5
   <223> Amidation
<400> 25
   Lys Leu Val Phe Phe
   1 5
<210> 26
   <211> 6
   <212> PRT
   <213> Artificial Sequence
<220>
   <223> Synthetic, All D peptides or peptidomimetics
<221> MOD_RES
   <222> 6
   <223> Amidation
<400> 26
   Lys Val Val Phe Phe Ala
   1 5
<210> 27
   <211> 7
   <212> PRT
   <213> Artificial Sequence
<220>
   <223> Synthetic, All D peptides or peptidomimetics
<400> 27
   Lys Leu Val Phe Phe Ala Glu
   1 5
<210> 28
   <211> 7
   <212> PRT
   <213> Artificial Sequence
<220>
   <223> Synthetic, All D peptides or peptidomimetics
<221> MOD_RES
   <222> 7
   <223> Amidation
<400> 28
   Lys Leu Val Phe Phe Ala Glu
   1 5
<210> 29
   <211> 10
   <212> PRT
   <213> Artificial Sequence
<220>
   <223> Synthetic, All D peptides or peptidomimetics
<400> 29
   His His Gln Lys Leu Val Phe Phe Ala Glu
   1 5 10
<210> 30
   <211> 3
   <212> PRT
   <213> Artificial Sequence
<220>
   <223> Synthetic, All D peptides or peptidomimetics
<400> 30
   Asp Asp Asp
   1
<210> 31
   <211> 6
   <212> PRT
   <213> Artificial Sequence
<220>
   <223> Synthetic, All D peptides or peptidomimetics
<400> 31
   Lys Val Asp Asp Gln Asp
   1 5
<210> 32
   <211> 4
   <212> PRT
   <213> Artificial Sequence
<220>
   <223> Synthetic, All D peptides or peptidomimetics
<400> 32
   His His Gln Lys
   1
<210> 33
   <211> 2
   <212> PRT
   <213> Artificial Sequence
<220>
   <223> Synthetic, All D peptides or peptidomimetics
<221> MOD_RES
   <222> 2
   <223> NHCH2CH2SO3H attached at the c-terminus.
<400> 33
   Phe Phe
   1
<210> 34
   <211> 2
   <212> PRT
   <213> Artificial Sequence
<220>
   <223> Synthetic, All D peptides or peptidomimetics
<221> MOD_RES
   <222> 2
   <223> NHCH2CH2CH2SO3H attached to the c-terminus
<400> 34
   Phe Phe
   1
<210> 35
   <211> 2
   <212> PRT
   <213> Artificial Sequence
<220>
   <223> Synthetic, All D peptides or peptidomimetics
<221> MOD_RES
   <222> 2
   <223> NHCH2CH2CH2CH2SO3H attached at the c-terminus
<400> 35
   Phe Phe
   1
<210> 36
   <211> 2
   <212> PRT
   <213> Artificial Sequence
<220>
   <223> Synthetic, All D peptides or peptidomimetics
<221> MOD_RES
   <222> 2
   <223> NHCH2CH2SO3H attached at the c-terminus
<400> 36
   Phe Tyr
   1
<210> 37
   <211> 2
   <212> PRT
   <213> Artificial Sequence
<220>
   <223> Synthetic, All D peptides or peptidomimetics
<221> MOD_RES
   <222> 2
   <223> NHCH2CH2CH2SO3H attached at the c-terminus.
<400> 37
   Phe Tyr
   1
<210> 38
   <211> 2
   <212> PRT
   <213> Artificial Sequence
<220>
   <223> Synthetic, All D peptides or peptidomimetics
<221> MOD_RES
   <222> 2
   <223> NHCH2CH2CH2CH2SO3H at the c-terminus
<400> 38
   Phe Tyr
   1
<210> 39
   <211> 2
   <212> PRT
   <213> Artificial Sequence
<220>
   <223> Synthetic, All D peptides or peptidomimetics
<221> MOD_RES
   <222> 1
   <223> H03SCH2CH2 attached at the n-terminus
<400> 39
   Phe Phe
   1
<210> 40
   <211> 2
   <212> PRT
   <213> Artificial Sequence
<220>
   <223> Synthetic, All D peptides or peptidomimetics
<221> MOD_RES
   <222> 1
   <223> H03SCH2CH2CH2 attached at the n-terminus
<400> 40
   Phe Phe
   1
<210> 41
   <211> 2
   <212> PRT
   <213> Artificial Sequence
<220>
   <223> Synthetic, All D peptides or peptidomimetics
<221> MOD_RES
   <222> 1
   <223> H03SCH2CH2CH2CH2 attached at the n-terminus
<400> 41
   Phe Phe
   1
<210> 42
   <211> 2
   <212> PRT
   <213> Artificial Sequence
<220>
   <223> Synthetic, All D peptides or peptidomimetics
<221> MOD_RES
   <222> 1
   <223> H03SCH2CH2 attached at the n-terminus
<400> 42
   Phe Tyr
   1
<210> 43
   <211> 2
   <212> PRT
   <213> Artificial Sequence
<220>
   <223> Synthetic, All D peptides or peptidomimetics
<221> MOD_RES
   <222> 1
   <223> H03SCH2CH2CH2 attached at the n-terminus
<400> 43
   Phe Tyr
   1
<210> 44
   <211> 2
   <212> PRT
   <213> Artificial Sequence
<220>
   <223> Synthetic, All D peptides or peptidomimetics
<221> MOD_RES
   <222> 1
   <223> H03SCH2CH2CH2CH2 attached at the n-terminus
<400> 44
   Phe Tyr
   1
<210> 45
   <211> 5
   <212> PRT
   <213> Artificial Sequence
<220>
   <223> Synthetic, All D peptides or peptidomimetics
<221> MOD_RES
   <222> 1
   <223> H03SCH2CH2 attached at the n-terminus
<400> 45
   Leu Val Phe Phe Ala
   1 5
<210> 46
   <211> 5
   <212> PRT
   <213> Artificial Sequence
<220>
   <223> Synthetic, All D peptides or peptidomimetics
<221> MOD_RES
   <222> 1
   <223> H03SCH2CH2CH2 attached at the n-terminus
<400> 46
   Leu Val Phe Phe Ala
   1 5
<210> 47
   <211> 5
   <212> PRT
   <213> Artificial Sequence
<220>
   <223> Synthetic, All D peptides or peptidomimetics
<221> MOD_RES
   <222> 1
   <223> H03SCH2CH2CH2CH2 attached at the n-terminus
<400> 47
   Leu Val Phe Phe Ala
   1 5
<210> 48
   <211> 5
   <212> PRT
   <213> Artificial Sequence
<220>
   <223> Synthetic, All D peptides or peptidomimetics
<221> MOD_RES
   <222> 5
   <223> NHCH2CH2SO3H attached at the c-terminus
<400> 48
   Leu Val Phe Phe Ala
   1 5
<210> 49
   <211> 5
   <212> PRT
   <213> Artificial Sequence
<220>
   <223> Synthetic, All D peptides or peptidomimetics
<221> MOD_RES
   <222> 5
   <223> NHCH2CH2CH2SO3H attached at the c-terminus
<400> 49
   Leu Val Phe Phe Ala
   1 5
<210> 50
   <211> 5
   <212> PRT
   <213> Artificial Sequence
<220>
   <223> Synthetic, All D peptides or peptidomimetics
<221> MOD_RES
   <222> 5
   <223> NHCH2CH2CH2CH2SO3H attached at the c-terminus
<400> 50
   Leu Val Phe Phe Ala
   1 5
<210> 51
   <211> 12
   <212> PRT
   <213> Artificial Sequence
<220>
   <223> Synthetic, positions 1-6 are L amino acids, and positions 7-12 are D amino acids
<400> 51
   Tyr Glu Val His His Gln Lys Leu Val Phe Phe Ala
   1 5 10
<210> 52
   <211> 13
   <212> PRT
   <213> Artificial Sequence
<220>
   <223> Synthetic, positions 1-6 are D amino acids, and positions 7-13 are L amino acids
<400> 52
   Lys Leu Val Phe Phe Ala Glu Asp Val Gly Ser Asn Lys
   1 5 10
<210> 53
   <211> 12
   <212> PRT
   <213> Artificial Sequence
<220>
   <223> Synthetic, All D peptides or peptidomimetics
<400> 53
   His His Gln Lys Leu Val Phe Phe Ala Glu Asp Val
   1 5 10
<210> 54
   <211> 13
   <212> PRT
   <213> Artificial Sequence
<220>
   <223> Synthetic, All D peptidomimetics
<400> 54
   Lys Leu Val Phe Phe Ala Glu Asp Val Gly Ser Asn Lys
   1 5 10
<210> 55
   <211> 17
   <212> PRT
   <213> Artificial Sequence
<220>
   <223> Synthetic, All D peptidominetics
<400> 55
   Arg His Asp Ser Gly Tyr Glu Val His His Gln Lys Leu Val Phe Phe
   1 5 10 15
   Ala
<210> 56
   <211> 6
   <212> PRT
   <213> Artificial Sequence
<220>
   <223> Synthetic, All D peptides or peptidomimetics
<221> MOD_RES
   <222> 6
   <223> Amidation
<400> 56
   Lys Leu Val Trp Phe Ala
   1 5
<210> 57
   <211> 6
   <212> PRT
   <213> Artificial Sequence
<220>
   <223> Synthetic, All D peptides or peptidomimetics
<221> MOD_RES
   <222> 6
   <223> Amidation
<400> 57
   Lys Leu Val Phe Trp Ala
   1 5
<210> 58
   <211> 6
   <212> PRT
   <213> Artificial Sequence
<220>
   <223> Synthetic, All D peptides or peptidomimetics
<221> MOD_RES
   <222> 6
   <223> Amidation
<400> 58
   Lys Leu Val Trp Trp Ala
   1 5
<210> 59
   <211> 6
   <212> PRT
   <213> Artificial Sequence
<220>
   <223> Synthetic, All D peptides or peptidomimetics
<221> MOD_RES
   <222> 6
   <223> Amidation
<400> 59
   Lys Leu Val Tyr Phe Ala
   1 5
<210> 60
   <211> 6
   <212> PRT
   <213> Artificial Sequence
<220>
   <223> Synthetic, All D peptides or peptidomimetics
<221> MOD_RES
   <222> 6
   <223> Amidation
<400> 60
   Lys Leu Val Phe Tyr Ala
   1 5
<210> 61
   <211> 6
   <212> PRT
   <213> Artificial Sequence
<220>
   <223> Synthetic, All D peptides or peptidomimetics
<221> MOD_RES
   <222> 6
   <223> Amidation
<400> 61
   Lys Leu Val Tyr Tyr Ala
   1 5
<210> 62
   <211> 6
   <212> PRT
   <213> Artificial Sequence
<220>
   <223> Synthetic, All D peptides or peptidomimetics
<221> MOD_RES
   <222> 4
   <223> Xaa at position 4 is thienylalanine
<221> MOD_RES
   <222> 6
   <223> Amidation
<400> 62
   Lys Leu Val Xaa Phe Ala
   1 5
<210> 63
   <211> 6
   <212> PRT
   <213> Artificial Sequence
<220>
   <223> Synthetic, All D peptides or peptidomimetics
<221> MOD_RES
   <222> 5
   <223> Xaa at position 5 is thienylalanine
<221> MOD_RES
   <222> 6
   <223> Amidation
<400> 63
   Lys Leu Val Phe Xaa Ala
   1 5
<210> 64
   <211> 6
   <212> PRT
   <213> Artificial Sequence
<220>
   <223> Synthetic, All D peptides or peptidomimetics
<221> MOD_RES
   <222> (4)...(5)
   <223> Xaa at positions 4 and 5 is thienylalanine
<221> MOD_RES
   <222> 6
   <223> Amidation
<400> 64
   Lys Leu Val Xaa Xaa Ala
   1 5
<210> 65
   <211> 6
   <212> PRT
   <213> Artificial Sequence
<220>
   <223> Synthetic, All D peptides or peptidomimetics
<221> MOD_RES
   <222> 4
   <223> Xaa at position 4 is cyclohexylalanine
<221> MOD_RES
   <222> 6
   <223> Amidation
<400> 65
   Lys Leu Val Xaa Phe Ala
   1 5
<210> 66
   <211> 6
   <212> PRT
   <213> Artificial Sequence
<220>
   <223> Synthetic, All D peptides or peptidomimetics
<221> MOD_RES
   <222> 5
   <223> Xaa at position 5 is cyclohexylalanine
<221> MOD_RES
   <222> 6
   <223> Amidation
<400> 66
   Lys Leu Val Phe Xaa Ala
   1 5
<210> 67
   <211> 6
   <212> PRT
   <213> Artificial Sequence
<220>
   <223> Synthetic, All D peptides or peptidomimetics
<221> MOD_RES
   <222> (4)...(5)
   <223> Xaa at positions 4 and 5 is cyclohexylalanine
<221> MOD_RES
   <222> 6
   <223> Amidation
<400> 67
   Lys Leu Val Xaa Xaa Ala
   1 5
<210> 68
   <211> 6
   <212> PRT
   <213> Artificial Sequence
<220>
   <223> Synthetic, All D peptides or peptidomimetics
<221> MOD_RES
   <222> 4
   <223> Xaa at position 4 is phenylglycine
<221> MOD_RES
   <222> 6
   <223> Amidation
<400> 68
   Lys Leu Val Xaa Phe Ala
   1 5
<210> 69
   <211> 6
   <212> PRT
   <213> Artificial Sequence
<220>
   <223> Synthetic, All D peptides or peptidomimetics
<221> MOD_RES
   <222> 5
   <223> Xaa at position 5 is phenylglycine
<221> MOD_RES
   <222> 6
   <223> Amidation
<400> 69
   Lys Leu Val Phe Xaa Ala
   1 5
<210> 70
   <211> 6
   <212> PRT
   <213> Artificial Sequence
<220>
   <223> Synthetic, All D peptides or peptidomimetics
<221> MOD_RES
   <222> (4)...(5)
   <223> Xaa at positions 4 and 5 is phenylglycine
<221> MOD_RES
   <222> 6
   <223> Amidation
<400> 70
   Lys Leu Val Xaa Xaa Ala
   1 5

## Description

### BACKGROUND OF THE INVENTION

The present invention relates to a new stereochemically based "non-self' antigen vaccine for the prevention and/or treatment of Alzheimer's and other amyloid related diseases.

Amyloidosis refers to a pathological condition characterized by the presence of amyloid fibers. Amyloid is a generic term referring to a group of diverse but specific protein deposits (intracellular and/or extracellular) which are seen in a number of different diseases. Though diverse in their occurrence, all amyloid deposits have common morphologic properties, stain with specific dyes (*e.g.*, Congo red), and have a characteristic red-green birefringent appearance in polarized light after staining. They also share common ultrastructural features and common x-ray diffraction and infrared spectra.

Amyloid-related diseases can either be restricted to one organ or spread to several organs. The first instance is referred to as "localized amyloidosis" while the second is referred to as "systemic amyloidosis".

Some amyloidotic diseases can be idiopathic, but most of these diseases appear as a complication of a previously existing disorder. For example, primary amyloidosis can appear without any other pathology or can follow plasma cell dyscrasia or multiple myeloma. Secondary amyloidosis is usually seen associated with chronic infection (such as tuberculosis) or chronic inflammation (such as rheumatoid arthritis). A familial form of secondary amyloidosis is also seen in Familial Mediterranean Fever (FMF). This familial type of amyloidosis, as one of the other types of familial amyloidosis, is genetically inherited and is found in specific population groups. In these two types of amyloidosis, deposits are found in several organs and are thus considered systemic amyloid diseases. Another type of systemic amyloidosis is found in long-term hemodialysis patients. In each of these cases, a different amyloidogenic protein is involved in amyloid deposition.

"Localized amyloidoses" are those that tend to involve a single organ system. Different amyloids are also characterized by the type of protein present in the deposit. For example, neurodegenerative diseases such as scrapie, bovine spongiform encephalitis, Creutzfeldt-Jakob disease and the like are characterized by the appearance and accumulation of a protease-resistant form of a prion protein (referred to as AScr or PrP-27) in the central nervous system. Similarly, Alzheimer's disease, another neurodegenerative disorder, is characterized by neuritic plaques and neurofibrillary tangles. In this case, the plaque and blood vessel amyloid is formed by the deposition of fibrillar Aβ amyloid protein. Other diseases such as adult-onset diabetes (Type II diabetes) are characterized by the localized accumulation of amyloid in the pancreas.

Once these amyloids have formed, there is no known, widely accepted therapy or treatment which significantly dissolves the deposits *in situ.*

Each amyloidogenic protein has the ability to organize into β-sheets and to form insoluble fibrils which get deposited extracellularly or intracellularly. Each amyloidogenic protein, although different in amino acid sequence, has the same property of forming fibrils and binding to other elements such as proteoglycan, amyloid P and complement component. Moreover, each amyloidogenic protein has amino acid sequences which, although different, will show similarities such as regions with the ability to bind to the glycosaminoglycan (GAG) portion of proteoglycan (referred to as the GAG binding site) as well as other regions which will promote β-sheet formation.

In specific cases, amyloidotic fibrils, once deposited, can become toxic to the surrounding cells. As per example, the Aβ fibrils organized as senile plaques have been shown to be associated with dead neuronal cells and microgliosis in patients with Alzheimer's disease. When tested *in vitro,* Aβ peptide was shown to be capable of triggering an activation process of microglia (brain macrophages), which would explain the presence of microgliosis and brain inflammation found in the brain of patients with Alzheimer's disease.

In another type of amyloidosis seen in patients with Type II diabetes, the amyloidogenic protein IAPP has been shown to induce β-islet cell toxicity *in vitro.* Hence, appearance of IAPP fibrils in the pancreas of Type II diabetic patients could contribute to the loss of the β islet cells (Langerhans) and organ dysfunction.

People suffering from Alzheimer's disease develop a progressive dementia in adulthood, accompanied by three main structural changes in the brain: diffuse loss of neurons in multiple parts of the brain; accumulation of intracellular protein deposits termed neurofibrillary tangles; and accumulation of extracellular protein deposits termed amyloid or senile plaques, surrounded by misshapen nerve terminals (dystrophic neurites). A main constituent of these amyloid plaques is the amyloid-β peptide (Aβ), a 40-42 amino-acid protein that is produced through cleavage of the β-amyloid precursor protein (APP). Although symptomatic treatments exist for Alzheimer's disease, this disease cannot be prevented nor cured at this time.

The use of a vaccine to treat Alzheimer's disease is possible in principle (Schenk, D. et al., (1999) Nature 400, 173-177). Schenk *et al.* show that, in a transgenic mouse model of brain amyloidosis (as seen in Alzheimer's disease), immunization with Aβ peptide inhibits the formation of amyloid plaques and the associated dystrophic neurites. In that study, a vaccine using the human aggregated all-L peptide as immunogen prevented the formation of β-amyloid plaque, astrogliosis and neuritic dystrophy in vaccinated transgenic mice.

The International patent application WO 00/68263 discloses antifibrillogenic agents for inhibiting amyloidosis and/or for cytoprotection for the treatment of amyloidosis disorders. These agents comprise a peptide having a sequence identified from the glycosaminoglycan binding region and the prot-prot interaction region of the amyloid protein. The peptide has at least one [D]-amino acid isomer substitution.

However, it is apparent that there are a number of drawbacks to using an endogenous protein as a vaccine (or a protein naturally present in the animal being vaccinated). Some of these drawbacks include:
- Possible development of autoimmune disease due to the generation of antibodies against "self' protein.
- Difficulty in eliciting an immune response due to the failure of the host immune system to break tolerance.
- Possible development of an acute inflammatory response in the brain due to antibody-mediated phagocytosis by microglia cells.
- Development of anti-idiotype antibodies.

### SUMMARY OF THE INVENTION

The present invention relates to a stereochemically based "non-self" antigen vaccine for the prevention and/or treatment of Alzheimer's and other amyloid related diseases. The present invention relates to a vaccine for the prevention and treatment of Alzheimer's and other amyloid related diseases, which overcomes the drawbacks associated with using naturally occurring peptides, proteins or immunogens, such as a fibril protein (e.g., beta Amyloid).

The present invention is to provide a
1. Use of a peptide for the manufacture of a medicament for preventing or treating an amyloid-related disease by inducing an immune response against amyloid-β in a mammal, wherein said peptide consists of an amino acid sequence as set forth in SEQ ID NO:8, or an amino acid sequence as set forth in SEQ ID NO:9, and wherein amino acids of said SEQ ID NO:8 or SEQ ID NO:9 consist entirely of [D]-amino acids.
2. In a preferred embodiment, the peptide for use in the present invention further comprises:
   (a) an N-terminal substituent selected from the group consisting of: hydrogen, lower alkyl group that is either acyclic or cyclic and has 1 to 8 carbon atoms, aromatic group, heterocyclic group, and acyl group; and
   (b) a C-terminal substituent selected from the group consisting of hydroxy, alkoxy, aryloxy, unsubstituted and substituted amino groups.

The term "amyloid related diseases" includes diseases associated with the accumulation of amyloid either in soluble or insoluble (plaque) forms, which can either be restricted to one organ, "localized amyloidosis", or spread to several organs, "systemic amyloidosis". Secondary amyloidosis may be associated with chronic infection (such as tuberculosis) or chronic inflammation (such as rheumatoid arthritis), including a familial form of secondary amyloidosis which is also seen in Familial Mediterranean Fever (FMF). Another type of systemic amyloidosis found in long-term hemodialysis patients. Localized forms of amyloidosis include, without limitation, diabetes type II and any related disorders thereof, neurodegenerative diseases such as scrapie, bovine spongiform encephalitis, Creutzfeldt-Jakob disease, Alzheimer's disease, Cerebral Amyloid Angiopathy, and prion protein related disorders.

A fibril peptide or protein for use in the present invention can be derived from a fibril precursor protein known to be associated with certain forms of amyloid diseases, as described herein. Such precursor proteins include, but are not limited to, Serum Amyloid A protein (ApoSAA), immunoglobulin light chain, immunoglobulin heavy chain, ApoAl, transthyretin, lysozyme, fibrinogen a chain, gelsolin, cystatin C, Amyloid beta protein precursor (β-APP), Beta₂ microglobulin, prion precursor protein (PrP), atrial natriuretic factor, keratin, islet amyloid polypeptide, a peptide hormone, and synuclein. Such precursors also include mutant proteins, protein fragments and proteolytic peptides of such precursors. In a preferred embodiment, the peptide is effective to induce an immune response directed against an epitope formed by an amyloidogenic protein or peptide, with respect to a fibril precursor protein. That is, as described in more detail herein, many fibril-forming peptides or proteins are fragments of such precursor proteins, such as those listed above. When such fragments are formed, such as by proteolytic cleavage, epitopes may be revealed that are not present on the precursor and are therefore not immunologically available to the immune system when the fragment is a part of the precursor protein.

The peptide is effective to induce an immune response directed against an epitope formed by an amyloidogenic protein or peptide. An amyloidogenic protein or peptide is synonymous with an amyloid peptide or protein, and encompasses a protein or peptide which is capable of forming fibrils, plaques, or amyloid deposits. The terms "Aβ," "Aβ peptide", "β-amyloid peptide" and "Amyloidβ" peptide are synonymous, and refer to one or more peptide compositions of about 38-43 amino acids derived from Beta Amyloid Precursor Protein (β-APP), as described herein. Disaggregated Aβ means soluble, monomeric and oligomeric peptide units of Aβ. One method to prepare monomeric Aβ is to dissolve lyophilized peptide in neat DMSO with sonication. The resulting solution is centrifuged to remove any insoluble particulates. Aggregated Aβ is a mixture of oligomers in which the monomeric units are held together by noncovalent bonds. Furthermore, APP⁶⁹⁵, APP⁷⁵¹, and APP⁷⁷⁰ refer, respectively, to the 695, 751, and 770 amino acid residue long polypeptides encoded by the human APP gene. See Kang et al., Nature 325, 773 (1987); Ponte et al., Nature 331, 525 (1988); and Kitaguchi et al., Nature 331, 530 (1988). Amino acids within the human amyloid precursor protein (APP) are assigned numbers according to the sequence of the APP77O isoform. Terms such as Aβ39, Aβ40, Aβ41, Aβ42 and Aβ43 refer, respectively, to an Aβ peptide containing amino acid residues 1-39, 1-40, 1-41, 1-42 and 1-43. Preferably, Aβ peptide contains amino acid residues 10-21. More preferably, the Aβ peptide contains amino acid residues 13-21.

Accordingly, a vaccine for use in the present invention is produced using a "non-self' peptide or protein synthesized from the unnatural D-configuration amino acids, to avoid the drawbacks of using "self' proteins. The peptides need not be aggregated to be operative or immunogenic, in contrast to the prior art vaccines. Thus, an "immunogenic peptide" or "immunogen" or "antigen" is a molecule that is capable of inducing an immunological response against itself upon administration to a patient, either in conjunction with, or in the absence of, an adjuvant. Such molecules include, for example, amyloidogenic peptides or fragments thereof conjugated to a carrier protein, such as keyhole limpet hemocyanin (KLH), C3d, polysaccharide, bovine serum albumin (BSA), Tetanus toxoid, heat shock protein (HSP), Ovalbumin or cholera toxin.

The term "immunological" or "immune" or "immunogenic" response refers to the development of a humoral (antibody mediated) and/or a cellular (mediated by antigen-specific T cells or their secretion products) response directed against an antigen in a vertebrate individual. Such a response can be an active response induced by administration of immunogen or a passive response induced by administration of antibodies or immune cells such as primed T-cells, B cells, macrophages NK or NKT cells, or primed dendritic cells which can act as antigen presenting cells. A cellular immune response is elicited by the presentation of polypeptide epitopes in association with Class I or Class II MHC molecules to activate antigen-specific CD4⁺ T helper cells and/or CD8⁺ cytotoxic T cells. The response may also involve activation of immune cells, such as monocytes, macrophages, NK cells, basophils, dendritic cells, astrocytes, microglia cells, eosinophils or other components of innate immunity. The presence of a cell-mediated immunological response can be determined by standard proliferation assays (CD4⁺ T cells) or CTL (cytotoxic T lymphocyte) assays known in the art. The relative contributions of humoral and cellular responses to the protective or therapeutic effect of an immunogen can be distinguished by separately isolating immunoglobulin (IgG) and T-cell fractions from an immunized first mammal and measuring a protective or therapeutic effect in a second subject.

The terms "polynucleotide" and "nucleic acid," as used interchangeably herein refer to a polymeric molecule having a backbone that supports bases capable of hydrogen bonding to typical polynucleotides, where the polymer backbone presents the bases in a manner to permit such hydrogen bonding in a sequence specific fashion between the polymeric molecule and a typical polynucleotide (e.g., single-stranded DNA). Such bases are typically inosine, adenosine, guanosine, cytosine, uracil and thymidine. Polymeric molecules include double and single stranded RNA and DNA, and backbone modifications thereof, for example, methylphosphonate linkages. The term "polypeptide" as used herein refers to a compound made up of a single chain of amino acid residues linked by peptide bonds. The term "protein" may be synonymous with the term "polypeptide" or may refer to a complex of two or more polypeptides. The term "peptide" also refers to a compound composed of amino acid residues linked by peptide bonds. Generally peptides are composed of 100 or fewer amino acids, while polypeptides or proteins have more than 100 amino acids. As used herein, the term "protein fragment" may also be read to mean a peptide.

Except as otherwise expressly defined herein, the abbreviations used herein for designating the amino acids and the protective groups are based on recommendations of the IUPAC-IUB Commission on Biochemical Nomenclature (Biochemistry, 1972, 11:1726-1732).

In another embodiment, there is provided a method for preventing and/or treating an amyloid-related disease in a subject, which features administering to the subject an antigenic amount of an all-D peptide which elicits production of antibodies against the all-D peptide, and elicits an immune response by the subject, therefore preventing fibrillogenesis, associated cellular toxicity and neurodegeneration, wherein the antibodies interact with at least one region of an amyloid protein, a fibril protein or another non-amyloid protein which induces amyloidosis. A "fibril peptide" or "fibril protein" refers to a monomeric or oligomeric or aggregated form of a protein or peptide that forms fibrils present in amyloid plaques. Examples of such peptides and proteins are provided herein. "Nonamyloid protein" containing formulations include, but are not limited to: compositions that produce immune responses against gelsolin fragments for treatment of hereditary systemic amyloidosis, mutant lysozyme protein (Alys), for treatment of a hereditary neuropathy, mutant alpha chain of fibrinogen (AfibA) for a non-neuropathic form of amyloidosis manifest as renal disease, and mutant cystatin C (Acys) for treatment of a form of hereditary cerebral angiopathy reported in Iceland. In addition, certain hereditary forms of prion disease (e.g., Creutzfeldt-Jacob disease (CJD), Gerstmann-Sträussler-Scheinker syndrome (GSS), and fatal familial insomnia (FFI)) are characterized by a mutant isoform of prion protein, PrP ^{Sc}. This protein can be used in therapeutic compositions for treatment and prevention of deposition of PrP plaques, in accordance with the present invention. These vaccines may be used in the prevention and/or treatment of amyloid related diseases, and in the manufacture of medicaments for preventing and/or treating amyloid-related diseases.

The term "antibody" or "immunoglobulin" is used to include intact antibodies and fragments thereof. An antibody can be a monoclonal or polyclonal antibody and can be made by recombinant techniques, collected from serum or ascites, or from hybridoma sources. Typically, fragments compete with the intact antibody from which they were derived for specific binding to an antigen fragment. Fragments are produced by recombinant DNA techniques, or by enzymatic or chemical cleavage of intact antibodies. Antigen binding fragments include Fab, Fab', F(ab')₂, Fv, and single-chain antibodies, further including separate heavy chains or light chains. Antibody fragments are produced by recombinant DNA techniques, or by enzymatic or chemical separation of intact immunoglobulins. The term "antibody" encompasses one or more immunoglobulin chains that are chemically conjugated to, or expressed as, fusion proteins with other proteins. The term "antibody" also includes bispecific antibodies. A bispecific or bifunctional antibody is an artificial hybrid antibody having two different heavy/light chain pairs and two different binding sites. Bispecific antibodies can be produced by a variety of methods including fusion of hybridomas or linking of Fab' fragments. See, e.g., Songsivilai & Lachmann, Clin. Exp. Immunol. 79:315-321 (1990); Kostelny et al., J. Immunol. 148, 1547-1553 (1992).

As used herein, "Single-chain Fv" or "sFv" antibody fragments comprise the VH and VL domains of antibody, wherein these domains are present in a single polypeptide chain. Preferably, the Fv polypeptide further comprises a polypeptide linker between the VH and VL domains which enables the sFv to form the desired structure for antigen binding. For a review of sFv *see,* Pluckthun, The Pharmacology of Monoclonal Antibodies. vol. 113. Rosenburg and Moore eds., Springer-Verlag, New York, pp. 269-315 (1994).

Specific binding between two entities means an affinity of at least 10⁶ M⁻¹, 10⁷ M⁻¹, 10⁸ M⁻¹, 10⁹M⁻¹, or 10¹⁰M⁻¹. Affinities greater than 10⁸ M⁻¹ are preferred.

As used herein, "Humanized" forms of non-human (e.g., rodent) antibodies are chimeric immunoglobulins, immunoglobulin chains or fragments thereof (such as Fv, Fab, Fab', F(ab')₂, or other antigen-binding subsequences of antibodies) which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat or rabbit having the desired specificity, affinity, and capacity. In some instances, framework region (FR) residues of the human immunoglobulin are replaced by corresponding non-human residues. Furthermore, humanized antibodies may comprise residues which are found neither in the recipient antibody nor in the donor antibody. These modifications are made to further refine and maximize antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details. see. Jones et al.. Nature, 3221:5222 525 (1986); Reichmarm et al., Nature, 332:323-329 (1988); and Presta, Curr. Op. Struct. Biol., 2:593-596 (1992).

The term "conjugate" or "conjugated to" is intended to refer to joined together, coupled, bonded and/or fused. A "protein conjugate" or "conjugated protein" is generally intended to refer to a compound of a protein with a non-protein. In addition to the amino acids, a "conjugated protein" contains permanently associated chemical components. The non-amino acid part of the conjugated protein is generally referred to as its prosthetic group. Conjugated proteins are classified on the basis of the chemical nature of their prosthetic groups (Lehninger Principles of Biochemistry, 3d ed. Worth Publishers 2000), which is incorporated by reference herein. In a conjugate vaccine, an antigenic molecule is covalently linked to a "carrier" protein or polypeptide. The linkage serves to increase the antigenicity of the conjugated molecule. Methods for forming conjugate vaccines from an antigenic molecule and a "carrier" protein or polypeptide are known in the art (Jacob, C. O, et al., Eur. J. Immunol. 16:1057-1062 (1986); Parker J. M. R. et al., In: Modem Approaches to Vaccines, Chanock, R. M. et al., eds, pp. 133-138, Cold Spring Harbor Laboratory, Cold Spring Harbor, N.Y. (1983); Zurawski, V. R, et al., J. Immunol. 121:122-129 (1978); Klipstein, F. A, et al., Infect. Immun. 37:550-557 (1982); Bessler, W. G, Immunobiol. 170:239-244 (1985); Posnett, D. N, et al., J. Biol. Chem. 263:1719-1725 (1988); Ghose, A. C, et al., Molec. Immunol. 25:223-230 (1988); all of which references are incorporated herein by reference). Conjugation of a protein and a polysaccharide can provide other advantageous results. For example, it has been found that protein-polysaccharide conjugates enhance the antibody response not only to the polysaccharide component, but also to the protein component. This effect is described, for example, in the dual conjugate patent application of Mond and Lees, U.S. Pas Nos. 5,955,079 and 5,585,100, both of which are incorporated herein by reference. This effect also is described in A. Lees, et al., "Enhanced Immunogenicity of Protein-Dextran Conjugates: I. Rapid Stimulation of Enhanced Antibody Responses to Poorly Immunogenic Molecules," Vaccine, Vol. 12, No. 13, (1994), pp. 1160-1166. This article is entirely incorporated herein by reference. Examples of carrier proteins include, but are not limited to keyhole limpet hemocyanin (KLH), C3d, polysaccharide, bovine serum albumin (BSA), Tetanus toxoid, heat shock protein (HSP), Ovalbumin or cholera toxin.

A "pharmaceutical composition" refers to a chemical or biological composition suitable for administration to a mammalian individual, preferably a human. Such compositions may be specifically formulated for administration via one or more of a number of routes, including but not limited to, oral, parenteral, intravenous, intraarterial, subcutaneous, intranasal, sublingual, intraspinal, intracerebroventricular, transdermal and the like. A "pharmaceutical excipient" or a "pharmaceutically acceptable excipient" is a carrier, usually a liquid, in which an active therapeutic peptide is formulated. The excipient generally does not provide any pharmacological activity to the formulation, though it may provide chemical and/or biological stability, release characteristics, and the like. Exemplary formulations can be found, for example, in Remington's Pharmaceutical Sciences, 19th Ed., Grennaro, A., Ed., 1995.

According to another related aspect, the invention includes the use of the peptides of the invention for the manufacture of a medicament for preventing or treating a disorder characterized by amyloid deposition in a mammalian subject. In accordance with this aspect of the invention, the subject is given a dosage of a peptide effective to produce an immune response against an amyloid peptide characteristic of the amyloid disorder from which the subject suffers. Essentially, the methods include administering pharmaceutical compositions containing immunogenic amyloid peptides specific to the disorder, such as those described above. Such methods are further characterized by their effectiveness in inducing immunogenic responses in the subject. According to a preferred embodiment, the method is effective to produce an immunological response that is characterized by a serum titer of at least 1:1000 with respect to the amyloid peptide against which the immunogenic peptide is directed. In yet a further preferred embodiment, the serum titer is at least 1:5000 with respect to the amyloid component. According to a related embodiment, the immune response is characterized by a serum amount of immunoreactivity corresponding to greater than about four times higher than a serum level of immunoreactivity measured in a pretreatment control serum sample. This latter characterization is particularly appropriate when serum immunoreactivity is measured by ELISA techniques, but can apply to any relative or absolute measurement of serum immunoreactivity. According to a preferred embodiment, the immunoreactivity is measured at a serum dilution of about 1:100 to 1:10,000 to determine antibody titer.

"Passive immunization" as used herein refers to the administration of antibodies, fragments thereof, or immune cells, i.e. T-cells B-cells, NK cells, NKT cells, dendritic cells, macrophages, basophils, monocytes, or components of the complement pathway, to an individual in order to confer immunity. Components of the complement pathway can be conjugated to proteins to enhance the inate immune response in combination with active or passive immunization. Immunoglobulins (Ig) obtained from human blood may contain antibodies to a variety of agents depending on the pool of human plasma used in preparation. Specific immunoglobulins are obtained from plasma from donors with high levels of antibodies to specific antigens, or donors immunized to produce such a response (Immunization, Cecil Textbook of Medicine, 19th ed. Vol. 1, W.B. Saunders Company 1992; Harrison's Principles of Internal Medicine, 14th ed, McGraw Hill, 1998). Humanized monoclonal antibodies to sequester amyloid-β peptide in plasma, brain and cerebrospinal fluid to prevent accumulation of the amyloid-β peptide within the brain and the cerebrovasculature is described in WO01/62801, which is incorporated herein by reference. EP0613007 describes antibodies having specificity for β-amyloid peptide which is predominantly in a β-sheet conformation. Such antibodies are useful for the invention a described herein.

The term "epitope" or "antigenic determinant" refers to a site on an antigen to which an antibody or antibody binding fragment specifically binds or to which B and/or T cells respond. B-cell epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, e.g., Epitope Mapping Protocols in Methods in Molecular Biology, Vol. 66, Glenn E. Morris, Ed. (1996). Antibodies that recognize the same epitope can be identified in a simple immunoassay showing the ability of one antibody to block the binding of another antibody to a target antigen. L-cells recognize continuous epitopes of about nine amino acids for CD8 cells or about 13-15 amino acids for CD4 cells. T cells that recognize the epitope can be identified by *in vitro* assays that measure antigen-dependent proliferation, as determined by ³H-thymidine incorporation by primed T cells in response to an epitope (Burke et al., J. Inf Dis. 170, 1110-19 (1994)), by antigen-dependent killing (cytotoxic T lymphocyte assay, Tigges et al., J. Immunol. 156, 3901-3910) or by cytokine secretion.

In a preferred embodiment of the present invention, the subject is a human being.

In yet another embodiment of the present invention, the amyloid related disease may be Alzheimer's disease.

In still another embodiment of the present invention, the amyloid related disease may be Cerebral Amyloid Angiopathy (CAA). In one aspect, altering serum Aβ levels in the mammal alters the levels of soluble Aβ or fibril Aβ in the mammal. In yet another aspect, altering serum Aβ levels in the mammal alters the levels of soluble or fibril Aβ levels in the brain of the mammal. In another aspect altering serum Aβ levels in the mammal favors the clearance of Aβ levels in the brain of a mammal.

The medicament can be used on both asymptomatic patients and those currently showing symptoms of disease.

Other features and advantages of the invention will be apparent from the following detailed description and claims.

### BRIEF DESCRIPTION OF THE DRAWING

FIG. 1 illustrates the targeted sites for the antigenic fragments.
FIG. 2 illustrates the effect of I mg/ml of polyclonal antibodies raised against D and L forms of Aβ(16-21) on fibrillogenesis.
FIG. 3 illustrates the effect of 0.5 mg/ml of polyclonal antibodies raised against D and L forms of Aβ(16-21) on fibrillogenesis.
FIGs. 4A to 4C illustrate electron micrographs showing the effect of anti-D KLVFFA peptide antibodies (FIG. 4B) and anti-L KLVFFA peptide antibodies (FIG. 4C) with respect to a control (FIG. 4A) on fibrillogenesis.
FIGs. 5A to 5D illustrate the immunohistochemistry of anti-D KLVFFA on aggregated Aβ peptide in brain sections of retrosplenial cortex (FIG. 5A) and parietal cortex (FIG. 5C) and the histochemistry (Thioflavin S assay) of anti-D KLVFFA on aggregated Aβ peptide in the same brain sections of retrospinal cortex (FIG. 5B) and parietal cortex (FIG. 5D).
FIGs. 6A to 6D illustrate the immunohistochemistry of anti-L KLVFFA antibodies on aggregated Aβ peptide in brain sections of parietal cortex (FIG. 6A) and entorrhinal cortex (FIG. 6C) and the histochemistry (Thioflavin S assay) of anti-L KLVFFA antibodies on aggregated Aβ peptide in the same brain sections of parietal cortex (FIG. 6B) and entorrhinal cortex (FIG. 6D).
FIG. 7 illustrates the response of rabbits to KLH-conjugated all-L and all-D KLVFFA.
FIGs. 8A and 8B illustrate the reduction of Aβ40 level, soluble and insoluble fraction in the brain of TgCRND8 mice immunized with D13-21-KLH.
Fig. 9 illustrates the reduction of Aβ42 level, soluble fraction in the brain of TgCRND8 mice immunized with D13-21-KLH.
FIGs. 10A and 10B illustrate the reduction of total Aβ level (Aβ40 and Aβ42) in the brain of TgCRND8 mice immunized with D13-21-KLH.

### DETAILED DESCRIPTION OF THE INVENTION

Amyloid diseases or amyloidoses include a number of disease states having a wide variety of outward symptoms. These disorders have in common the presence of abnormal extracellular deposits of protein fibrils, known as "amyloid deposits" or "amyloid plaques" that are usually about 10-100nm in diameter and are localized to specific organs or tissue regions. Such plaques are composed primarily of a naturally occurring soluble protein or peptide which aggregate in insoluble fibrillar deposits. These insoluble deposits are composed of generally lateral aggregates of fibrils that are approximately 10-15 nm in diameter. Amyloid fibrils produce a characteristic apple green birefringence in polarized light, when stained with Congo Red dye.

The peptides or proteins forming the plaque deposits are often produced from a larger precursor protein. More specifically, the pathogenesis of amyloid fibril deposits generally involves proteolytic cleavage of an "abnormal" precursor protein into fragments. These fragments generally aggregate into anti-parallel β-pleated sheets; however, certain undegraded forms of precursor protein have been reported to aggregate and form fibrils in familial amyloid polyneuropathy (variant transthyretin fibrils) and dialysis-related amyloidosis (β₂ microglobulin fibrils) (Tan, S.Y. and Pepys, M.B. Amyloidosis. Histopathology 25:403-414 (1994)). Specifically, the Aβ (16-21) site is known to play an important role in initiating the harmful process of Aβ peptide amyloidogenesis. It is also known that when these peptides are made from D-amino acids, they retain their ability to interact with the natural all-L-homologous sequence, thereby preventing amyloidogenesis. Other amyloid proteins which may be used in the present invention include, without limitation, the beta sheet regions of IAPP (e.g. 24-29, all-D), β2-microglobulin, amyloid A protein, and prion-related proteins.

The disorders are traditionally classified on the basis of the major fibril components forming the plaque deposits, as discussed below. However, without being restricted to theory, there is evidence supporting Aβ can be transported back and forth between the brain and the blood (Ghersi-Egea, J., et al., J. Neurochem., 67:880-883, (1996)) and that Aβ in plaques (insoluble or fibrillar Aβ is in equilibrium with soluble Aβ (Kawarabayashi, T., et al., J. Neurosci. 21:372-381, (2001), both incorporated herein by reference in their entirety. Thus, the identification of a disorder based on the major fibril component is provided for convenience, and amelioration of the disease can be effectuated according to the compositions and methods described herein by targeting either the fibrillar Aβ or the soluble Aβ. The targeting of either the fibrillar Aβ or the soluble Aβ can be achieved by active or passive immunization as described herein and in PCT publications WO 01/62801, WO 01/90182, WO 01/18169, WO 00/77178, WO 00/72880, WO 00/72876, WO 99/60024 and WO 99/27944, each incorporated by reference in its entirety. Levels of targeted Aβ, specifically levels of the soluble Aβ can be determined by methods known in the art and disclosed herein (*see,* U.S. patents 5,766,846, 5,837,672, and 5,593,846, each incorporated by reference in their entirety). These levels can be monitored during the course of prophylactic or therapeutic treatments. A therapeutic endpoint is reached when the disease state is reduced.

### Amyloid Diseases

The present invention is based on the discovery that amyloid diseases can be treated by administering peptides that serve to stimulate an immune response against a component or components of the various disease-specific amyloid deposits. The sections below serve to exemplify major forms of amyloidosis and are not intended to limit the invention.

### AA (reactive) Amyloidosis

Generally, AA amyloidosis is a manifestation of a number of diseases that provoke a sustained acute phase response. Such diseases include chronic inflammatory disorders, chronic local or systemic microbial infections, and malignant neoplasms.

AA fibrils are generally composed of 8000 dalton fragments (AA peptide or protein) formed by proteolytic cleavage of serum amyloid A protein (ApoSAA), a circulating apolipoprotein which is present in HDL complexes and which is synthesized in hepatocytes in response to such cytokines as IL-1, IL-6 and TNF. Deposition can be widespread in the body, with a preference for parenchymal organs. The spleen is usually a deposition site, and the kidneys may also be affected. Deposition is also common in the heart and gastrointestinal tract.

AA amyloid diseases include, but are not limited to inflammatory diseases, such as rheumatoid arthritis, juvenile chronic arthritis, ankylosing spondylitis, psoriasis, psoriatic arthropathy, Reiter's syndrome, Adult Still's disease, Behcet's syndrome, and Crohn's disease. AA deposits are also produced as a result of chronic microbial infections, such as leprosy, tuberculosis, bronchiectasis, decubitus ulcers, chronic pyelonephritis, osteomyelitis, and Whipple's disease. Certain malignant neoplasms can also result in AA fibril amyloid deposits. These include such conditions as Hodgkin's lymphoma, renal carcinoma, carcinomas of gut, lung and urogenital tract, basal cell carcinoma, and hairy cell leukemia.

### AL Amyloidoses

AL amyloid deposition is generally associated with almost any dyscrasia of the B lymphocyte lineage, ranging from malignancy of plasma cells (multiple myeloma) to benign monoclonal gammopathy. At times, the presence of amyloid deposits may be a primary indicator of the underlying dyscrasia.

Fibrils of AL amyloid deposits are composed of monoclonal immunoglobulin light chains or fragments thereof. More specifically, the fragments are derived from the N-terminal region of the light chain (kappa or lambda) and contain all or part of the variable (V_{L}) domain thereof. Deposits generally occur in the mesenchymal tissues, causing peripheral and autonomic neuropathy, carpal tunnel syndrome, macroglossia, restrictive cardiomyopathy, arthropathy of large joints, immune dyscrasias, myelomas, as well as occult dyscrasias. However, it should be noted that almost any tissue, particularly visceral organs such as the heart, may be involved.

### Hereditary Systemic Amyloidoses

There are many forms of hereditary systemic amyloidoses. Although they are relatively rare conditions, adult onset of symptoms and their inheritance patterns (usually autosomal dominant) lead to persistence of such disorders in the general population. Generally, the syndromes are attributable to point mutations in the precursor protein leading to production of variant asnyloidogenic peptides or proteins. Table 1 summarizes the fibril composition of exemplary forms of these disorders.

All compounds are listed for illustrative purposes only.

**Table 1**

| ***Fibril Peptide*/*Protein*** | ***Genetic variant*** | ***Clinical Syndrome*** |
|---|---|---|
| Transthyretin and fragments (ATTR) | Met30, many others | Familial amyloid polyneuropahty (FAP), (Mainly peripheral nerves) |
| Transthyretin and fragments (ATTR) | Thr45, Ala60, Ser84, Met 111, Ile122 | Cardiac involvement predominant without neuropathy |
| N-terminal fragment of Apolipoprotein A1 (apoAI) | Arg26 | Familial amyloid polyneurophathy (FAP), (mainly peripheral nerves) |
| N-terminal fragment of Apoliproprotein A1 (AapoAI) | Arg26, Arg50, Arg 60, others | Ostertag-type, non-neuropathic (predominantly visceral involvement) |
| Lysozyme (Alys) | Thr56, His67 | Ostertag-type, non-neuropathic (predominantly visceral involvement) |
| Fibrogen ∀ chain fragment | Leu554, Val 526 | Cranial neuropathy with lattic corneal dystrophy |
| Gelsolin fragment (Agel) | Asn187, Tyr187 | Cranial neuropathy with lattice cornealk dystrophy |
| Cystatin C fragment | Glu68 | Hereditary cerebral hemmorrhage (cerebral amyloid angiopathy) - Icelandic type |
| β-amyloid protein (aβ) derived from Amyloid Precursor Protein (APP) | Gln693 | Hereditary cerebral hemmorrhage (cerebral amyloid angiopathy) - Dutch type |
| β-amyloid protein (aβ) derived from Amyloid Precursor Protein (APP) | Ile717, Phe717, Gly717 | Familial Alzheimer's Disease |
| β-amyloid protein (aβ) derived from Amyloid Precursor Protein (APP) | Asn670,Leu671 | Familial Dementia - probably Alzheimer's Disease |
| Prion Protein (PrP) derived from Prp precursor protein 51-91 insert | Leu102, Val167, Asn178, Lys200 | Familial Creutzfeldt-Jakob disease; Gerstmann-Sträussler-Scheinker syndrome (hereditary spongiform encephalopathies, prion diseases) |
| AA derived from Serum amyloid A protein (ApoSAA) | | Familial Mediterranean fever, predominant renal involvement (autosomal recessive) |
| AA derived from Serum amyloid A protein (ApoSAA) | | Muckle-Well's syndrome, nephropathy, deafness, urticaria, limb pain |
| Unknown | | Cardiomyophathy with persistent atrial standstill |
| Unknown | | Cutaneous deposits (bullous, papular, pustulodermal) |

| | | |
|---|---|---|
| *Data derived from Tan & Pepys, 1994, supra. | | |

The data provided in Table 1 are exemplary and are not intended to limit the scope of the invention. For example, more than 40 separate point mutations in the transthyretin gene have been described, all of which give rise to clinically similar forms of familial amyloid polyneuropathy.

Transthyretin (TTR) is a 14 kilodalton protein that is also sometimes referred to as prealbumin. It is produced by the liver and choroid plexus, and it functions in transporting thyroid hormones and vitamin A. At least 50 variant forms of the protein, each characterized by a single amino acid change, are responsible for various forms of familial amyloid polyneuropathy. For example, substitution of proline for leucine at position 55 results in a particularly progressive form of neuropathy; substitution of methionine for leucine at position 111 resulted in a severe cardiopathy in Danish patients. Amyloid deposits isolated from heart tissue of patients with systemic amyloidosis have revealed that the deposits are composed of a heterogeneous mixture of TTR and fragments thereof, collectively referred to as ATTR, the full length sequences of which have been characterized. ATTR fibril components can be extracted from such plaques and their structure and sequence determined according to the methods known in the art (e.g., Gustavsson, A., et al., Laboratory Invest. 73: 703-708, 1995; Kametani, F., et al., Biochem. Biophys. Res. Commun. 125: 622-628, 1984; Pras, M., et al., PNAS 80: 539-42, 1983).

Persons having point mutations in the molecule apolipoprotein Al (e.g., Gly→ Arg26; Trp 4→ Arg50; Leu→ 4 Arg60) exhibit a form of amyloidosis ("Östertag type") characterized by deposits of the protein apolipoprotein AI or fragments thereof (AApoAI). These patients have low levels of high density lipoprotein (HDL) and present with a peripheral neuropathy or renal failure.

A mutation in the alpha chain of the enzyme lysozyme (e.g., Ile→Thr56 or Asp→His57) is the basis of another form of Östertag-type non-neuropathic hereditary amyloid reported in English families. Here, fibrils of the mutant lysozyme protein (Alys) are deposited, and patients generally exhibit impaired renal function. This protein, unlike most of the fibril-forming proteins described herein, is usually present in whole (unfragmented) form (Benson, M.D., et al. CIBA Fdn. Symp. 199: 104-131, 1996).

β-amyloid peptide (Aβ) is a 39-43 amino acid peptide derived by proteolysis from a large protein known as β Amyloid Precursor protein (βAPP). Mutations in βAPP result in familial forms of Alzheimer's disease, Down's syndrome and/or senile dementia, characterized by cerebral deposition of plaques composed of Aβ fibrils and other components, which are described in further detail below. Known mutations in APP associated with Alzheimer's disease occur proximate to the cleavage sites of β or gamma-secretase, or within Aβ. For example, position 717 is proximate to the site of gamma-secretase cleavage of APP in its processing to Aβ, and positions 670/671 are proximate to the site of β-secretase cleavage. Mutations at any of these residues may result in Alzheimer's disease, presumably by-causing an increase the amount of the 42/43 amino acid form of Aβ generated from APP. The structure and sequence of Aβ peptides of various lengths are well known in the art. Such peptides can be made according to methods known in the art (e.g., Glenner and Wong, Biochem Biophys. Res. Comm. 129: 885-890, 1984; Glenner and Wong, Biochem Biophys. Res. Comm. 122: 113 1-1135,1984). In addition, various forms of the peptides are commercially available.

Synuclein is a synapse-associated protein that resembles an apolipoprotein and is abundant in neuronal cytosol and presynaptic terminals. A peptide fragment derived from alpha-synuclein, termed NAC, is also a component of amyloid plaques of Alzheimer's disease. (Clayton, et al., 1998). This component also serves as a target for immunologically-based treatments of the present invention, as detailed below.

Gelsolin is a calcium binding protein that binds to fragments and actin filaments. Mutations at position 187 (e.g., Asp→ Asn; Asp→ Tyr) of the protein result in a form of hereditary systemic amyloidosis, usually found in patients from Finland, as well as persons of Dutch or Japanese origin. In afflicted individuals, fibrils formed from gelsolin fragments (Agel), usually consist of amino acids 173-243 (68 kDa carboxyterminal fragment) and are deposited in blood vessels and basement membranes, resulting in corneal dystrophy and cranial neuropathy which progresses to peripheral neuropathy, dystrophic skin changes and deposition in other organs. (Kangas, H., et al. Human Mol. Genet. 5(9): 1237-1243, 1996).

Other mutated proteins, such as mutant alpha chain of fibrinogen (AfibA) and mutant cystatin C (Acys) also form fibrils and produce characteristic hereditary disorders. AfibA fibrils form deposits characteristic of a nonneuropathic hereditary amyloid with renal disease; Acys deposits are characteristic of a hereditary cerebral amyloid angiopathy reported in Iceland. (Isselbacher, Ct at., Harrison's Principles of Internal Medicine, McGraw-Hill, San Francisco, 1995; Benson, *et al., supra.*). In at least some cases, patients with cerebral amyloid angiopathy (CAA) have been shown to have amyloid fibrils containing a non-mutant form of cystatin C in conjunction with beta protein. (Nagai, A., et al. Molec. Chem. Neuropathol. 33: 63-78, 1998).

Certain forms of prion disease are now considered to be heritable, accounting for up to 15% of cases, which were previously thought to be predominantly infectious in nature. (Baldwin, et al., in Research Advances in Alzheimer's Disease and Related Disorders, John Wiley and Sons, New York, 1995). In such prion disorders, patients develop plaques composed of abnormal isoforms of the normal prion protein (PrP^{Sc}). A predominant mutant isoform, PrP^{Sc}, also referred to as AScr, differs from the normal cellular protein in its resistance to protease degradation, insolubility after detergent extraction, deposition in secondary lysosomes, post-translational synthesis, and high β-pleated sheet content. Genetic linkage has been established for at least five mutations resulting in Creutzfeldt-Jacob disease (CJD), Gerstmann-Sträussler-Scheinker syndrome (GSS), and fatal familial insomnia (FFI). (Baldwin) Methods for extracting fibril peptides from scrapie fibrils, determining sequences and making such peptides are known in the art. (e.g., Beekes, M., et al. J. Gen. Virol. 76: 2567-76, 1995).

For example, one form of GSS has been linked to a PrP mutation at codon 102, while telencephalic GSS segregates with a mutation at codon 117. Mutations at codons 198 and 217 result in a form of GSS in which neuritic plaques characteristic of Alzheimer's disease contain PrP instead of Aβ peptide. Certain forms of familial CJD have been associated with mutations at codons 200 and 210; mutations at codons 129 and 178 have been found in both familial CJD and FFI. (Baldwin, *supra*).

### Senile Systemic Amyloidosis

Amyloid deposition, either systemic or focal, increases with age. For example, fibrils of wild type transthyretin (TTR) are commonly found in the heart tissue of elderly individuals. These may be asymptomatic, clinically silent, or may result in heart failure. Asymptomatic fibrillar focal deposits may also occur in the brain (Aβ), corpora amylacea of the prostate (β₂ microglobulin), joints and seminal vesicles.

### Cerebral Amyloidosis

Local deposition of amyloid is common in the brain, particularly in elderly individuals. The most frequent type of amyloid in the brain is composed primarily of Aβ peptide fibrils, resulting in dementia or sporadic (non-hereditary) Alzheimer's disease. In fact, the incidence of sporadic Alzheimer's disease greatly exceeds forms shown to be hereditary. Fibril peptides forming these plaques are very similar to those described above, with reference to hereditary forms of Alzheimer's disease (AD).

### Dialysis-related Amyloidosis

Plaques composed of β₂ microglobulin (β₂M) fibrils commonly develop in patients receiving long term hemodialysis or peritoneal dialysis. β₂ microglobulin is a 11.8 kilodalton polypeptide and is the light chain of Class I MHC antigens, which are present on all nucleated cells. Under normal circumstances, it is continuously shed from cell membranes and is normally filtered by the kidney. Failure of clearance, such as in the case of impaired renal function, leads to deposition in the kidney and other sites (primarily in-collagen-rich tissues of the joints). Unlike other fibril proteins, β₂M molecules are generally present in unfragmented form in the fibrils. (Benson, *supra*).

### Hormone-derived Amyloidoses

Endocrine organs may harbor amyloid deposits, particularly in aged individuals. Hormone-secreting tumors may also contain hormone-derived amyloid plaques, the fibrils of which are made up of polypeptide hormones such as calcitonin (medullary carcinoma of the thyroid), islet amyloid polypeptide (amylin; occurring in most patients with Type II diabetes), and atrial natriuretic peptide (isolated atrial amyloidosis). Sequences and structures of these proteins are well known in the art.

### Miscellaneous Amyloidoses

There are a variety of other forms of amyloid disease that are normally manifest as localized deposits of amyloid. In general, these diseases are probably the result of the localized production and/or lack of catabolism of specific fibril precursors or a predisposition of a particular tissue (such as the joint) for fibril deposition. Examples of such idiopathic deposition include nodular AL amyloid, cutaneous amyloid, endocrine amyloid, and tumor-related amyloid.

### Pharmaceutical Compositions

The pharmaceutical compositions of the present invention are directed to vaccines prepared from fibril peptides that have 100% of their amino acid residues in the dextro form (D-isomers). The vaccines for use in the present invention are prepared from all D-peptides consisting of an amino acid sequence as set forth in SEQ ID NO: 8 or in SEQ ID NO: 9. The vaccines are believed to elicit an immune response in the host or to produce antibodies that recognize the naturally occurring target. As used herein, "all-D" refers to peptides having 100% D-configuration amino acids.

The vaccine according to the present invention is able to prevent the development of brain amyloidosis through two possible scenarios: 1) the effect of anti-Aβ antibodies at the site of amyloid deposition, and 2) the systemic effect of the high circulatory anti-Aβ level on the plasmatic Aβ concentrations.

Specifically, elevated plasma anti-Aβ antibody levels may act systemically by decreasing normal Aβ plasma levels, thereby creating a systemic imbalance in the normal Aβ levels. Such an imbalance could lead to activation of the mechanism responsible for clearing Aβ from the brain and into the periphery, in order to re-establish the normal balance between brain and plasma Aβ levels.

Accordingly, this possibility could be exploited by determining the effect of active or passive immunization on plasma and brain Aβ40 levels at different timepoints following such immunization. Aβ-immunization can also exert a systemic protective effect against the development of brain amyloidosis. The ratio of Aβ levels in plasma and brain should remain constant in immunized transgenic animals, while it should decrease in control animals. Additionally, B-cell or bone marrow cell transfer from immunized to naïve transgenic animals should have the same effect as passive immunization using anti-Aβ antibodies.

Furthermore, the vaccine for use in the present invention does not present the drawbacks of using "self' proteins and does not need to be aggregated to induce an immune response. For example, the antibodies raised against the all-D-Aβ(16-21) peptide can be expected to recognize the all-L-Aβ(16-21) peptide sequence. Pharmaceutical compositions of the present invention may include, in addition to the immunogenic peptide(s), an effective amount of an adjuvant and/or an excipient. Pharmaceutically effective and useful adjuvants and excipients are well known in the art, and are described in more detail below.

According to the present invention, compositions capable of eliciting or providing an immune response directed to certain components of amyloid plaques are effective to treat or prevent development of amyloid diseases. In particular, according to the invention provided herein, it is possible to prevent progression of, ameliorate the symptoms of, and/or reduce the amyloid deposition process in afflicted individuals, when an immunostimulatory dose of an anti-amyloid peptide, or corresponding anti-amyloid immune peptide, is administered to the patient. This section describes exemplary anti-amyloid peptides that produce active, as well as passive, immune responses to amyloid protein and provides exemplary data showing the effect of treatment using such compositions on amyloid brain concentration.

### Anti-Amyloid Peptides: Antibodies, Analogs and Fragments ofAmyloid Proteins

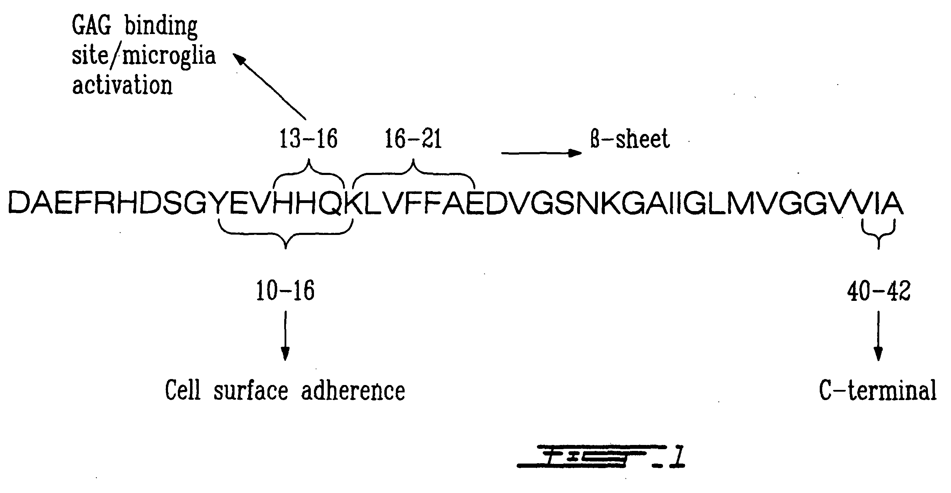

Generally, anti-amyloid peptides of the invention are composed of a specific plaque component, preferably an amyloid or amyloidogenic component, which is usually a characteristic protein, peptide, or fragment thereof. The human forms of Aβ are referred to as Aβ39, Aβ40, Aβ41, Aβ42 and Aβ43. The sequences of these peptides and their relationship to the APP precursor are illustrated by Fig. 1 of Hardy et al., TINS 20, 155-158 (1997). For example, Aβ42 has the sequence:
H₂N-Asp-Ala-Glu-Phe-Arg-His-Asp-Ser-Gly-Tyr-Glu-Val-His-His-Gln-Lys-Leu-Val-Phe-Phe-Ala-Glu-Asp-Val-Gly-Ser-Asn-Lys-Gly-Ala-Ile-Ile-Gly-Leu-Met-Met-Val-Gly-Gly-Val-Val-IIe-Ala-OH (SEQ ID NO:1)

Aβ41, Aβ40 and Aβ39 differ from Aβ42 by the omission of Ala, Ala-Ile and Ala-lie-Val respectively from the C-terminal end. Aβ43 differs from Aβ42 by the presence of a threonine residue at the C-terminus.

Immunogenic fragments of Aβ are advantageous relative to the intact molecule in the present methods for several reasons. First, because only certain epitopes within Aβ induce a useful immunogenic response for treatment of Alzheimer's disease, an equal dosage of mass of a fragment containing such epitopes provides a greater molar concentration of the useful immunogenic epitopes than a dosage of intact Aβ. Second, certain immunogenic fragments of Aβ generate an immunogenic response against amyloid protein without generating a significant immunogenic response against APP protein from which Aβ derives. Third, fragments of Aβ are simpler to manufacture than intact Aβ due to their shorter size. Fourth, fragments of Aβ do not aggregate in the same manner as intact Aβ, simplifying preparation of pharmaceutical compositions and administration thereof. Fifth, full-length Aβ is toxic to cells, but fragments are not, and Aβ aggregates could act as seeds to accelerate plaque deposition, but non-aggregated fragments would not. Sixth, Aβ has the unusual property that it can fix and activate both classical and alternate complement cascades. In particular, it binds to Clq and ultimately to C3bi. This association facilitates binding to macrophages leading to activation of B cells. In addition, C3bi breaks down further and then binds to CR2 on B cells in a T cell dependent manner leading to a 10,000 fold increase in activation of these cells. This mechanism causes Aβ to generate an immune response in excess of that of other antigens (Bradt et al., Complement-dependent Proinflammatory Properties of the Alzheimer's Disease β-Peptide, J. Exp. Med. Vol.188, pp. 431-438 (1998); Thornton et al., Clin. Exp. Immunol. Vol. 104, pp. 531-7 (1996); Jacquier-Sarlin et al., Immunology vol. 84, pp. 164-70 (1995).
isoaspartic acid. Fragments and analogs can be screened for prophylactic or therapeutic efficacy in transgenic animal models in comparison with untreated or placebo controls as described below.

Aβ, its fragments and analogs can be synthesized by solid phase peptide synthesis or recombinant expression, or can be obtained from natural sources. Automatic peptide synthesizers are commercially available from numerous suppliers, such as Applied Biosystems, Foster City, California. Recombinant expression can be in bacteria, such as E. coli, yeast, insect cells or mammalian cells. Procedures for recombinant expression are described by Sambrook et al., Molecular Cloning: A Laboratory Manual (C.S.H.P. Press, NY 2d ed., 1989). Some forms of Aβ peptide are also available commercially (e.g., American Peptides Company, Inc., Sunnyvale, CA and California Peptide Research, Inc. Napa, CA).

The peptides for use in the present invention can be administered in associated or multimeric form or in dissociated form. The peptides for use in the present invention produce or induce an immune response against an amyloid protein. Antibodies may also bind the soluble form in the periphery to act as a sink and drive Aβ from the brain into the periphery, facilitating the clearance of Aβ from the brain by either modulating Aβ equilibrium from the CNS to the periphery or forming a stable complex with Aβ in the periphery. Induction of an immune response can be active, as when an immunogen is administered to induce antibodies or T-cells reactive with the component in a patient, or passive, as when an antibody is administered that itself binds to the amyloid peptide in the patient.

A general class of anti-amyloid peptides consists of peptides that are derived from amyloid proteins. As mentioned above, the hallmark of amyloid diseases is the deposition in an organ or organ of amyloid plaques consisting mainly of fibrils, which, in turn, are composed of characteristic amyloid proteins or peptides. Table 1 summarizes exemplary fibril-forming proteins that are characteristic of various amyloid diseases.

Other formulations for treating hereditary forms of amyloidosis, discussed above, include compositions that produce immune responses against gelsolin fragments for treatment of hereditary systemic amyloidosis, mutant lysozyme protein (Alys), for treatment of a hereditary neuropathy, mutant alpha chain of fibrinogen (AfibA) for a non-neuropathic form of amyloidosis manifest as renal disease, and mutant cystatin C (Acys) for treatment of a form of hereditary cerebral angiopathy reported in Iceland. In addition, certain hereditary forms of prion disease (e.g., Creutzfeldt-Jacob disease (CJD), Gerstmann-Sträussler-Scheinker syndrome (GSS), and fatal familial insomnia (FFI)) are characterised by a mutant isoform ofprion protein, PrP ^{Sc}. This protein can be used in therapeutic compositions for treatment and prevention of deposition of PrP plaques, in accordance with the present invention.

As discussed above, amyloid deposition, either systemic or focal, is also associated with aging. It is a further aspect of the present invention that such deposition can be prevented or treated by administering to susceptible individuals compositions consisting of one or more proteins associated with such aging. Thus, plaques composed of ATTR derived from wild type TTR are frequently found in heart tissue of the elderly. Similarly, certain elderly individuals may develop asymptomatic fibrillar focal deposits of Aβ in their brains; Aβ peptide treatment, as detailed herein may be warranted in such individuals. By way of further example, but not limitation, there are a number of additional, non-hereditary forms of amyloid disease that are candidates for treatment methods of the present invention. β₂ microglobulin fibrillar plaques commonly develop in patients receiving long term hemodialysis or peritoneal dialysis. Such patients may be treated with therapeutic compositions directed to β₂ microglobulin or, more preferably, immunogenic epitopes thereof, in accordance with the present invention.

The peptides of the present invention can be synthesized by solid phase peptide synthesis or recombinant expression, according to standard methods well known in the art, or can be obtained from natural sources. Exemplary fibril compositions, methods of extraction of fibrils, sequences of fibril peptide or protein components are provided by many of the references cited in conjunction with the descriptions of the specific fibril components provided herein. Additionally, other compositions, methods of extracting and determining sequences are known in the art available to persons desiring to make and use such compositions. Automatic peptide synthesizers may be used to make such compositions and are commercially available from numerous manufacturers, such as Applied Biosystems (Perkin Elmer; Foster City, California), and procedures for preparing synthetic peptides are known in the art. Recombinant expression can be in bacteria, such as E. coli, yeast, insect cells or mammalian cells; alternatively, proteins can be produced using cell free *in vitro* translation systems known in the art. Procedures for recombinant expression are described by Sambrook et al., Molecular Cloning: A Laboratory Manual (C.S.H.P. Press, NY 2d ed., 1989). Certain peptides and proteins are also available commercially; for example, some forms of Aβ peptide are available from suppliers such as American Peptides Company, Inc., Sunnyvale, California, and California Peptide Research, Inc. Napa, California.

### Immunization Procedures

The elicited antibodies present in the host having received the vaccine of the present invention bind at the Aβ(16-21) epitope or other epitopes such as Aβ(10-21), Aβ(13-21). AB (10-22), AB)13-22), AB (16-22) and the N- or C-terminal region of Aβ and have the ability to prevent or reverse amyloidogenesis. The vaccine of the present invention causes the generation of effective antiamyloidogenic antibodies in the vaccinated host.

A suggested immunization procedure is as follows:
a) prepare a vaccine from an all-D peptide of the present invention;
b) immunize a host with the vaccine to generate an antibody in the host with a binding site capable of preventing fibrillogenesis, associated cellular toxicity and neurodegeneration.

Suitable pharmaceutically acceptable carriers include, without limitation, any non-immunogenic pharmaceutical adjuvants suitable for oral, parenteral, nasal, mucosal, transdermal, intravascular (IV), intraarterial (IA), intramuscular (IM), and subcutaneous (SC) administration routes, such as phosphate buffer saline (PBS).

The pharmaceutical carriers may contain a vehicle, which carries antigens to antigen-presenting cells. Examples of vehicles are liposomes, immune-stimulating complexes, microfluidized squalene-in-water emulsions, microspheres which may be composed of poly(lactic/glycolic) acid (PLGA). Particulates of defined dimensions (<5 micron) include, without limitation, oil-in-water microemulsion (MF59) and polymeric microparticules.

The carriers of the present invention may also include chemical and genetic adjuvants or immunostimulants to augment immune responses or to increase the antigenicity of immunogens. These adjuvants or immunostimulants exert their immunomodulatory properties through several mechanisms such as lymphoid cell recruitment, cytokine induction, and the facilitation of DNA entry into cells. Cytokine adjuvants include, without limitation, granulocyte-macrophage colony-stimulating factor, interleukin-12, GM-CSF, synthetic muramyl dipeptide analog or monophosphoryl lipid A. Other chemical adjuvants or immunostimulants include, without limitation, lactic acid bacteria, Al(OH)₃, muramyl dipeptides and saponins. Examples of microbial adjuvants include, without limitation, CpG motifs, Freund's, muramyl dipeptide, LPS derivatives, heat shock protein (HSP), lipid A derivative, polysaccharides, cholera toxin, killed Bordetella pertussis and LT (lymphotoxin *E. coli*). Examples of non-microbial adjuvants include, without limitation, aluminum salt, alum, mineral oil, iscoms, liposomes, virosomes, archaeosomes, transfersomes, niosomes, cochleates, proteosomes, calcium phosphate, DDA (dimethyldioctadecylammonium bromide), cytokines, hormones and C3d. Such adjuvants are well understood in the art.

The peptide may be coupled to a carrier that will modulate the half-life of the circulating peptide. This will allow control of the period of protection. The peptide-carrier may also be emulsified in an adjuvant and administrated by a standard immunization route.

The compositions and methods of the present invention can be administered therapeutically or prophylactically to treat diseases associated with amyloid-β fibril formation, aggregation or deposition. The compositions and methods of the invention may also act to ameliorate the course of an amyloid-β related disease using any of the following mechanisms, which are meant to be illustrative and not limiting: by slowing the rate of amyloid-β fibril formation or deposition; by lessening the degree of amyloid-β deposition; by inhibiting, preventing or reducing amyloid-β fibril formation; by inhibiting neurodegeneration or cellular toxicity induced by amyloid-β; by inhibiting amyloid-β induced inflammation; by enhancing the clearance of amyloid-β from the brain; or by reducing the levels of the amyloid-β40 or amyloid-β42 peptides in the brain or the plasma.

In a preferred embodiment, the method is used to treat Alzheimer's disease (e.g. sporadic or familial AD). The method can also be used prophylactically or therapeutically to treat other clinical occurrences of amyloid-β deposition, such as in Down's syndrome individuals and in patients with Cerebral Amyloid Angiopathy, hereditary cerebral amyloid angiopathy, hereditary cerebral hemorrhage or hemorrhagic stroke.

Additionally, abnormal accumulation of APP (β-amyloid precursor protein) and of amyloid-β protein in muscle fibers has been implicated in the pathology of sporadic inclusion body myositis (IBM) (Askanas, V. et al. (1996) Proc. Natl. Acad. Sci. USA 93: 1314-1319; Askanas, V. et al. (1995) Current Opinion in Rheumatology 7: 486-496). Accordingly, the compounds of the invention can be used prophylactically or therapeutically in the treatment of disorders in which amyloid-β protein is abnormally deposited at non-neurological locations, such as treatment of IBM by delivery of the compounds to muscle fibers.

The vaccine for use in the present invention will, for the most part, be administered parenterally, such as intravascularly (IV), intraarterially (IA), intramuscularly (IM), subcutaneously (SC), transdermally or the like. In some instances, administration may be oral, nasal, rectal, transdermal or aerosol, where the nature of the vaccine allows for transfer to the vascular system. Usually a single injection will be employed although more than one injection may be used, if desired. Typically the primary immunization will be followed by multiple boosts with an interval of a few weeks, using the same antigen or a further modified antigen if desired. The adjuvants or immunostimulants may also be identical or different if desired. The vaccine may be administered by any convenient means, including syringe, trocar, catheter, or the like. Preferably, the administration will be intravascular, where the site of introduction is not critical to this invention, preferably at a site where there is rapid blood flow, *e.g.*, intravenously, in a peripheral or central vein. Other routes may find use where the administration is coupled with slow release techniques or a protective matrix.

The use of the vaccine of the present invention in preventing and/or treating Alzheimer's disease and other amyloid related diseases can be validated by raising antibodies against the corresponding all-D peptide and testing them to see if they can effectively inhibit or prevent the fibrillogenesis of the natural amyloid peptide (all-L)

The compounds used to prepare vaccines in accordance with the present invention are selected from peptides consisting of an amino acid sequence as set forth in SEQ ID NO: 8 or SEQ ID NO: 9, consisting entirely of [D]-amino acids.

The peptide may further comprise:
(a) an N-terminal substitue selected from the group consisting of:
   ■ hydrogen;
   ■ lower alky groups, that is either acyclic or cyclic having 1 to 8 carbon atoms;
   ■ aromatic groups;
   ■ heterocyclic groups; and
   ■ acyl groups, *e.g.,* alkylcarbonyl, arylcarbonyl, sulfonyl and phosphonyl groups; and
(b) a C-terminal substituent, hydroxy, alkoxy, aryloxy, unsubstituted or substituted amino groups.

R' and R" may be identical or different; the alkyl or aryl group of R' and R" may further be substituted with organic functionalities selected from the group of halides (F, Cl, Br, and I), hydroxyl, alkoxyl, aryloxyl, hydroxycarbonyl, alkoxylcarbonyl, aryloxycarbonyl, carbamyl, unsubstituted or substituted amino, sulfo or alkyloxysulfonyl, phosphono or alkoxyphosphonyl, and the like.

The term "alkyl" includes saturated aliphatic groups, including straight-chain alkyl groups (*e.g.*, methyl, ethyl, propyl, butyl, pentyl, hexyl, heptyl, and octyl,), branched-chain alkyl groups (*e.g.*, isopropyl, tert-butyl, isobutyl), cycloalkyl-(*e.g.*, alicyclic) groups (*e.g.*, cyclopropyl, cyclopentyl, cyclohexyl, cycloheptyl, cyclooctyl), alkyl substituted cycloalkyl groups, and cycloalkyl substituted alkyl groups. "Alkyl" further includes alkyl groups which have oxygen, nitrogen, sulfur or phosphorous atoms replacing one or more hydrocarbon backbone carbon atoms. In certain embodiments, a straight chain or branched chain alkyl has six or fewer carbon atoms in its backbone (e.g., C₁-C₆ for straight chain, C₃-C₆ for branched chain), and more preferably four or fewer. Likewise, preferred cycloalkyls have from three to eight carbon atoms in their ring structure, and more preferably have five or six carbons in the ring structure. "C₁-C₆" includes alkyl groups containing one to six carbon atoms.

The term "alkyl" also includes both "unsubstituted alkyls" and "substituted alkyls", the latter of which refers to alkyl moieties having substituents replacing a hydrogen on one or more carbons of the hydrocarbon backbone. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Cycloalkyls can be further substituted, *e.g.*, with the substituents described above. An "alkylaryl" or an "aralkyl" moiety is an alkyl substituted with an aryl (*e.g.*, phenylmethyl (benzyl)). "Alkyl" also includes the side chains of natural and unnatural amino acids.

As used herein, "acyl groups" include compounds and moieties which contain the acyl radical (CH₃CO-) or a carbonyl group. "Substituted acyl" includes acyl groups where one or more of the hydrogen atoms are replaced by for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, phosphonyl, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, sulfonyl nitro, trifluoromethyl, cyano, azido, heterocyclyl, alkylaryl, or an aromatic or heteroaromatic moiety, i.e. arylcarbonyl.

The term "heterocyclic group" includes closed ring structures, *e.g.*, 3- to 10-, or 4- to 7-membered rings, which include one or more heteroatoms. Heterocyclyl groups can be saturated or unsaturated and include pyrrolidine, oxolane, thiolane, piperidine, piperazine, morpholine, lactones, lactams such as azetidinones and pyrrolidinones, sultams, sultones, and the like. The heterocyclic ring can be substituted at one or more positions with such substituents as described above, as for example, halogen, hydroxyl, alkykarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, sulfonato, sulfamoyl, sulfonamido, nitro, trifluoromethyl, cyano, azido, heterocyclyl, or an aromatic or heteroaromatic moiety.

As used herein, the term "aromatic groups" is intended to encompass "aryl" substituents. "Aryl" includes groups with aromaticity, including 5- and 6-membered single-ring aromatic groups that may include from zero to four heteroatoms, as well as multicyclic systems with at least one aromatic ring. Examples of aryl groups include benzene, phenyl, pyrrole, furan, thiophene, thiazole, isothiazole, imidazole, triazole, tetrazole, pyrazole, oxazole, isooxazole, pyridine, pyrazine, pyridazine, and pyrimidine, and the like. Furthermore, the term "aryl" includes multicyclic aryl groups, *e.g.*, tricyclic, bicyclic, e.g., naphthalene, benzoxazole, benzodioxazole, benzothiazole, benzoimidazole, benzothiophene, methylenedioxyphenyl, quinoline, isoquinoline, napthridine, indole, benzofuran, purine, benzofuran, deazapurine, or indolizine. Those aryl groups having heteroatoms in the ring structure may also be referred to as "aryl heterocycles", "heterocycles," "heteroaryls" or "heteroaromatics". The aromatic ring can be substituted at one or more ring positions with such substituents as described above, as for example, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, aralkylaminocarbonyl, alkenylaminocarbonyl, alkylcarbonyl, arylcarbonyl, aralkylcarbonyl, alkenylcarbonyl, alkoxycarbonyl, aminocarbonyl, alkylthiocarbonyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Aryl groups can also be fused or bridged with alicyclic or heterocyclic rings which are not aromatic so as to form a multicyclic system (e.g., tetralin, methylenedioxyphenyl).

Where a functional group is an acid, its pharmaceutically acceptable salt or ester is in the scope of this invention. Where a functional group is a base, its pharmaceutically acceptable salt is in the scope of this invention.

In one embodiment, P is a peptide capable of interacting with at least one region of an amyloid protein or fibril.

In a further embodiment, the preferred compounds may be coupled with a carrier that will modulate the biodistribution, immunogenic property and the half-life of the compounds.

The following are exemplary compounds for preparing vaccines for preventing or treating Alzheimer's disease and other amyloid related diseases and are listed for illustrative purposes only:

| | |
|---|---|
| SEQ ID NO: 1 | Aβ (1-42, all-D) |
| | DAEFRHDSGYEVHHQKLVFFAEDVGSNKGAIIGLMV GGVVIA |
| | |
| SEQ ID NO: 2 | Aβ (1-40, all-D) |
| | DAEFRHDSGYEVHHQKLVFFAEDVGSNKGAJIIGLMV GGVV |
| | |
| SEQ ID NO: 3 | Aβ (1-35, all-D) |
| | DAEFRHDSGYEVHHQKLVFFAEDVGSNKGAIIGLM |
| | |
| SEQ ID NO: 4 | Aβ (1-28, all-D) |
| | DAEFRHDSGYEVHHQKLVFFAEDVGSNK |
| | |
| SEQ ID NO: 5 | Aβ (1-7, all-D) |
| | DAEFRHD |
| | |
| SEQ ID NO: 6 | Aβ (10-16, all-D) |
| | YEVHHQK |
| | |
| SEQ ID NO:7 | Aβ (16-21, all-D) |
| | KLVFFA |
| | |
| SEQ ID NO: 8 | Aβ (10-21, all-D) |
| | YEVHHQKLVFFA |
| | |
| SEQ ID NO: 9 | Aβ (13-21, all-D) |
| | HHQKLVFFA |
| | |
| SEQ ID NO: 10 | Aβ (36-42, all-D) |
| | VGGVVIA |
| | |
| SEQ ID NO: 11 | Lys-Ile-Val-Phe-Phe-Ala (all-D) |
| | |
| SEQ ID NO: 12 | Lys-Lys-Leu-Val-Phe-Phe-Ala(all-D) |
| | |
| SEQ ID NO: 13 | Lys-Phe-Val-Phe-Phe-Ala (all-D) |
| | |
| SEQ ID NO: 14 | Ala-Phe-Phe-Val-Leu-Lys (all-D) |
| | |
| SEQ ID NO: 15 | Lys-Leu-Val-Phe (all-D) |
| | |
| SEQ ID NO: 16 | Lys-Ala-Val-Phe-Phe-Ala (all-D) |
| | |
| SEQ ID NO: 17 | Lys-Leu-Val-Phe-Phe (all-D) |
| | |
| SEQ ID NO: 18 | Lys-Leu-Val-Phe-Phe-Ala-NH₂ (all-D) |
| | |
| SEQ ID NO: 19 | Lys-Ile-Val-Phe-Phe-Ala-NH₂ (all-D) |
| | |
| SEQ ID NO: 20 | Lys-Leu-Val-Phe-Phe-Ala-NH₂ (all-D) |
| | |
| SEQ ID NO: 21 | Lys-Phe-Val-Phe-Phe-Ala-NH₂ (all-D) |
| | |
| SEQ ID NO: 22 | Ala-Phe-Phe-Val-Leu-Lys-NH₂ (all-D) |
| | |
| SEQ ID NO: 23 | Lys-Leu-Val-Phe-NH₂ (all-D) |
| | |
| SEQ ID NO: 24 | Lys-Ala-Val-Phe-Phe-Ala-NH₂ (all-D) |
| | |
| SEQ ID NO: 25 | Lys-Leu-Val-Phe-Phe-NH₂ (all-D) |
| | |
| SEQ ID NO: 26 | Lys-Val-Val-Phe-Phe-Ala-NH₂ (all-D) |
| | |
| SEQ ID NO: 27 | Lys-Leu-Val-Phe-Phe-Ala-Glu (all-D) |
| | |
| SEQ ID NO: 28 | Lys-Leu-Val-Phe-Phe-Ala-Glu-NH₂ (all-D) |
| | |
| SEQ ID NO: 29 | His-His-Gln-Lys-Leu-Val-Phe-Phe-Ala-Glu (all-D) |
| | |
| SEQ ID NO: 30 | Asp-Asp-Asp (all-D) |
| | |
| SEQ ID NO: 31 | Lys-Val-Asp-Asp-Gln-Asp (all-D) |
| | |
| SEQ ID NO: 32 | His-His-Gln-Lys (all-D) |
| | |
| SEQ ID NO: 33 | Phe-Phe-NH-CH₂CH₂SO₃H (all-D) |
| | |
| SEQ ID NO: 34 | Phe-Phe-NH-CH₂CH₂CH₂SO₃H (all-D) |
| | |
| SEQ ID NO: 35 | Phe-Phe-NH-CH₂CH₂CH₂CH₂SO₃H (all-D) |
| | |
| SEQ ID NO: 36 | Phe-Tyr-NH-CH₂CH₂SO₃H (all-D) |
| | |
| SEQ ID NO: 37 | Phe-Tyr-NH-CH₂CH₂CH₂SO₃H (all-D) |
| | |
| SEQ ID NO: 38 | Phe-Tyr-NH-CH₂CH₂CH₂CH₂SO₃H (all-D) |
| | |
| SEQ ID NO: 39 | HO₃SCH₂CH₂-Phe-Phe (all-D) |
| | |
| SEQ ID NO: 40 | HO₃SCH₂CH₂CH₂-Phe-Phe (all-D) |
| | |
| SEQ ID NO: 41 | HO₃SCH₂CH₂CH₂CH₂-Phe-Phe (all-D) |
| | |
| SEQ ID NO: 42 | HO₃SCH₂CH₂-Phe-Tyr (all-D) |
| | |
| SEQ ID NO: 43 | HO₃SCH₂CH₂CH₂-Phe-Tyr (all-D) |
| | |
| SEQ ID NO: 44 | HO₃SCH₂CH₂CH₂CH₂-Phe-Tyr (all-D) |
| | |
| SEQ ID NO: 45 | HO₃SCH₂CH₂-Leu-Val-Phe-Phe-Ala (all-D) |
| | |
| SEQ ID NO: 46 | HO₃SCH₂CH₂CH₂-Leu-Val-Phe-Phe-Ala (all-D) |
| | |
| SEQ ID NO: 47 | HO₃SCH₂CH₂CH₂CH₂-Leu-Val-Phe-Phe-Ala (all-D) |
| | |
| SEQ ID NO: 48 | Leu-Val-Phe-Phe-Ala-NH-CH₂CH₂SO₃H (all-D) |
| | |
| SEQ ID NO: 49 | Leu-Val-Phe-Phe-Ala-NH-CH₂CH₂CH₂SO₃H (all-D) |
| | |
| SEQ ID NO: 50 | Leu-Val-Phe-Phe-Ala-NH-CH₂CH₂CH₂CH₂SO₃H (all-D) |
| | |
| SEQ ID NO: 51 | Aβ([L] 10-15[D] 16-21) |
| | Y[L]-E[L]-V[L]-H[L]-H[L]-Q[L]-K[D]-L[D]-V[D]-F[D]-F[D]-A[D] |
| | |
| SEQ ID NO: 52 | Aβ ({D] 16-21 [L] 22-28) |
| | K[D]-L[D]-V[D]-F[D]-F[D]-A[D]-E[L]-D[L]-V[L]-G[L]-S[L]-N[L]-K[L] |
| | |
| SEQ ID NO: 53 | His-His-Gln-Lys-Leu-Val-Phe-Phe-Ala-Glu-Asp-Val |
| | (all-D) |
| | |
| SEQ ID NO: 54 | Lys-Leu-Val-Phe-Phe-Ala-Glu-Asp-Val-Gly-Ser-Asn-Lys |
| | (all-D) |
| SEQ ID NO: 55 | |
| | Arg-His-Asp-Ser-Gly-Tyr-Glu-Val-His-His-Gln-Lys-Leu-Val-Phe-Phe-Ala (all-D) |

The following are exemplary compounds derived from compound 18 (all-D KLVFFA-NH₂; SEQ ID NO: 18) by substituting one or two amino acid residue(s) with other amino acids and are listed for illustrative purposes only:

| | |
|---|---|
| SEQ ID NO: 56 | Lys-Leu-Val-Trp-Phe-Ala-NH₂ (all-D) |
| | |
| SEQ ID NO: 57 | Lys-Leu-Val-Phe-Trp-Ala- NH₂ (all-D) |
| | |
| SEQ ID NO: 58 | Lys-Leu-Val-Trp-Trp-Ala- NH₂ (all-D) |
| | |
| SEQ ID NO: 59 | Lys-Leu-Val-Tyr-Phe-Ala- NH₂ (all-D) |
| | |
| SEQ ID NO: 60 | Lys-Leu-Val-Phe-Tyr-Ala- NH₂ (all-D) |
| | |
| SEQ ID NO: 61 | Lys-Leu-Val-Tyr-Tyr-Ala- NH₂ (all-D) |
| | |
| SEQ ID NO: 62 | Lys-Leu-Val-Thi-Phe-Ala- NH₂ (all-D) |
| | |
| SEQ ID NO: 63 | Lys-Leu-Val-Phe-Thi-Ala- NH₂ (all-D) |
| | |
| SEQ ID NO: 64 | Lys-Leu-Val-Thi-Thi-Ala- NH₂ (all-D) |
| | |
| SEQ ID NO: 65 | Lys-Leu-Val-Cha-Phe-Ala- NH₂ (all-D) |
| | |
| SEQ ID NO: 66 | Lys-Leu-Val-Phe-Cha-Ala- NH₂ (all-D) |
| | |
| SEQ ID NO: 67 | Lys-Leu-Val-Cha-Cha-Ala- NH₂ (all-D) |
| | |
| SEQ ID NO: 68 | Lys-Leu-Val-Pgly-Phe-Ala- NH₂ (all-D) |
| | |
| SEQ ID NO: 69 | Lys-Leu-Val-Phe-Pgly-Ala- NH₂ (all-D) |
| | |
| SEQ ID NO: 70 | Lys-Leu-Val-Pgly-Pgly-Ala- NH₂ (all-D). |

For the above compounds, the terms Thi, Cha and Pgly are intended to mean thienylalanine, cyclohexylalanine and phenylglycine, respectively.

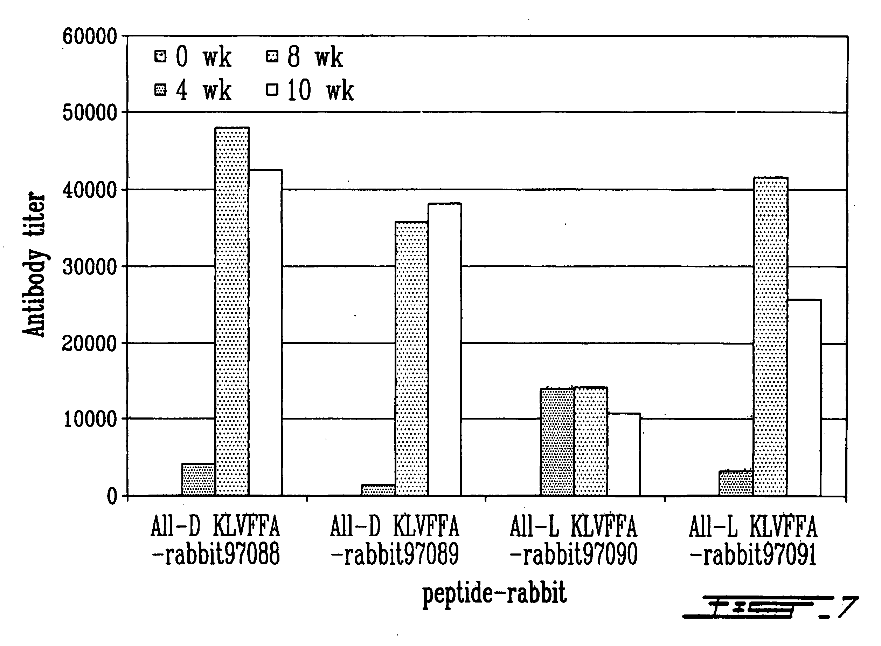

Rabbits were immunized with all-D or all-L KLVFFA. Results of the antibody titers obtained are shown in FIG. 7. As seen in FIG. 7, the vaccine of the present invention causes production of antibodies.

In another experiment, adult TgCRND8 mice were immunized with all-D or all-L KLH-amyloid-β fragment ([L]-amyloid-β 13-21 and [D] amyloid-β 13-21). Results of the antibody titers is shown in Table 2.

**Table 2**

| **KLH-amyloid-β fragment** | **Mean Plasma Antibody** |
|---|---|
| | **Titer (inverse) in TgCRND8** |
| [L] amyloid-β 13-21 | <100 |
| [D] amyloid-β 13-21 | 11,000 |

The present invention encompasses various types of immune responses triggered using the vaccine of the present invention, *e.g.*, amyloid therapies using the vaccine approach.

In accordance with the present invention, there is also provided a vaccine which triggers a preferential TH-2 response or a TH-1 response, according to the type of immunization used. By inducing a TH-2 response, anti-inflammatory cytokine production such as IL-4, II-10 and TGF-β, as well as the production of IgG 1 and IgG 2b antibody classes, are favored. Such type of response would be preferred, as a major inflammatory response in the brain of the patients with AD would be avoided. On the other hand, with a preferred TH-1 response, a pro-inflammatory response with a production of inflammatory cytokines such as IL-1, IL-6, TNF and IFN gamma would be favored. This type of response would more likely trigger activation of the macrophage population. These macrophages would then phagocytose any particulate deposits (such as plaques) via a complement-activated process as well as via an antibody-mediated process. This approach would be beneficial to clear already organized senile plaques and prevent the formation of new fibrillary deposits. However this response would also be accompanied by an inflammatory response which would be detrimental to the host.

Both approaches (*i.e.* TH-1 and TH-2) are of value. The antigen used could be the peptides which contain regions responsible for cellular adherence, *i.e.,* region 10-16, regions responsible for the GAG binding site, *i.e.,* 13-16, regions responsible for the β sheet, *i.e.* 16-21, or regions specific for the C-terminus, *i.e.* 40-42. These peptides could be presented in such a way that either a preferential TH-1 or TH-2 response is obtained, depending on the type of adjuvant used, or depending on the route of administration of the vaccine. For example, a mucosal immunization via nasal administration is possible, since it is known that such a route of administration would favor a TH-2 response.

The present invention will be more readily understood by referring to the following examples, which are given to illustrate the invention rather than to limit its scope.

### EXAMPLE I

An *in vitro* validation procedure to test the effectiveness of all-D peptide vaccines derived from fibrillogenic amyloid proteins was performed in rabbits or mice to demonstrate that antibodies can be raised against Aβ 16-21 (all-D) (see FIG. 7). The antibodies produced were tested to prove that they effectively prevent the fibrillogenesis of natural Aβ (1-40, all-L) *in vitro.* Standard assays for fibrillogenesis were used to evaluate activity, such as those based on Thioflavine T, circular dichroism and solubility.

This approach was also used to establish which areas of the Aβ peptide are most effective when used in the form of all-D peptides to prepare antifibrillogenic vaccines. The experiments were performed as follows:
a) rabbits or mice were immunized with a series of overlapping all-D peptides generated from the Aβ (1-42) sequence, *e.g.*, Aβ (16-21), Aβ (10-21), Aβ (13-21), etc.
b) antisera were prepared from the immunized rabbits or mice.
c) these antisera were tested to see which parts of the Aβ sequence produce antisera that most effectively prevent fibrillogenesis in the standard assays for fibrillogenesis mentioned above.

### EXAMPLE II

### Effect of Antibodies Against D- and L-Aβ (16-21) Peptide Vaccine on Fibrillogenesis

A validation procedure to test anti-fibrillogenic activity of antibodies raised against D- and L- Aβ (16-21) peptide was performed.

Rabbits were immunized with D- or L-Aβ (16-21) peptide. Antibodies raised were tested for their antifibrillogenic activities by ThT assay and by electron microscopy (EM).

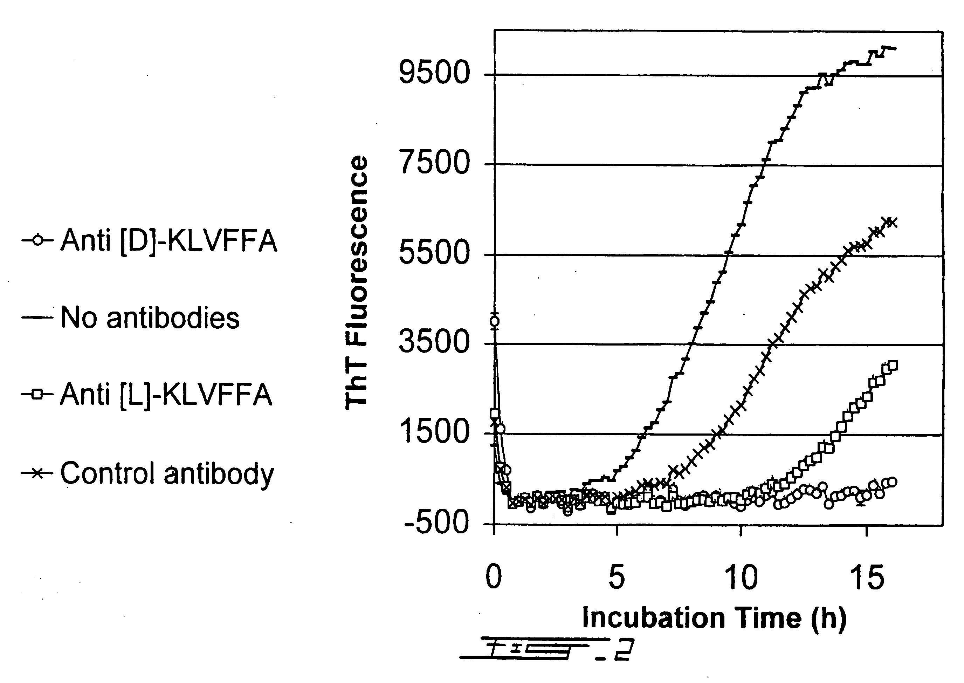

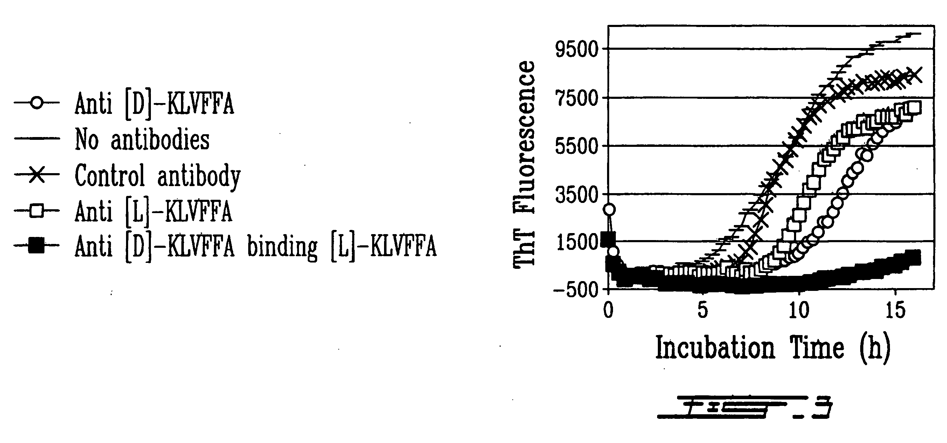

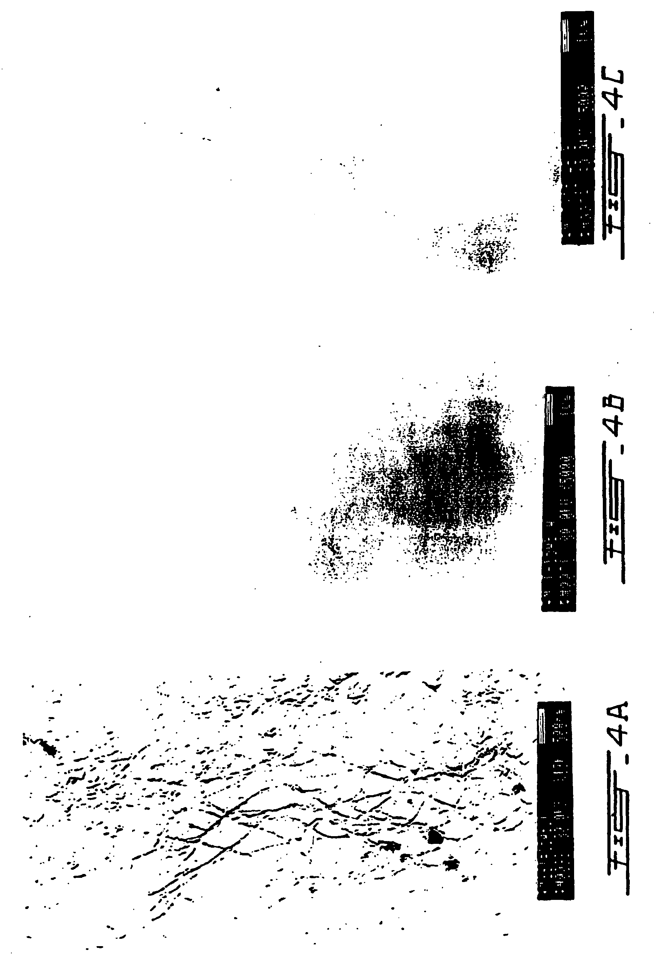

Antibodies raised against the D- and L- forms of KLVFFA were capable of blocking the fibrillogenesis process as seen either by the Thioflavin T assay (ThT) (FIGs. 2 and 3) and by EM (FIGs. 4A to 4C). In the ThT assay, fibril formation is monitored by the increase in fluorescence with time. As seen in the Figures, the antibodies were capable of inhibiting such an increase in fluorescence, proving that these antibodies were inhibiting fibrillogenesis.

As can be seen in these figures (FIGs. 2 to 4), antibodies raised against the D-peptide have a better anti-fibrillogenic activity than anti-L antibodies.

These results were also confirmed by EM (FIGs. 4A to 4C) where both anti-D and anti-L KLVFFA peptide blocked the fibril formation when compared to control (FIG. 4A). Moreover, again the anti-D peptide has a greater anti-fibrillogenic activity (FIG. 4B) than the anti-L peptide (FIG. 4C). This goes along with the ThT assay where the decrease in fluorescence was greater with the anti-D peptide antibody than with the anti-L peptide antibody.

### EXAMPLE III

### Antibody Binding Assay

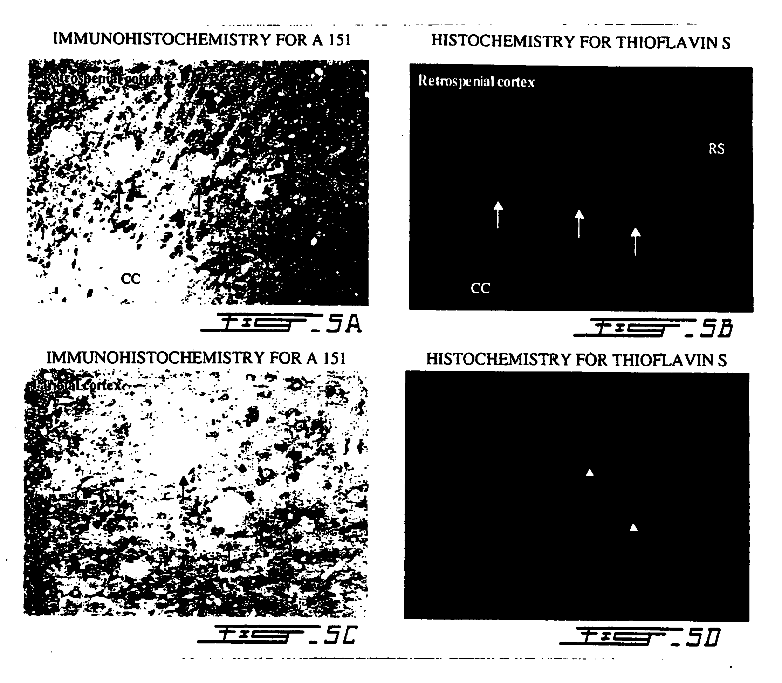

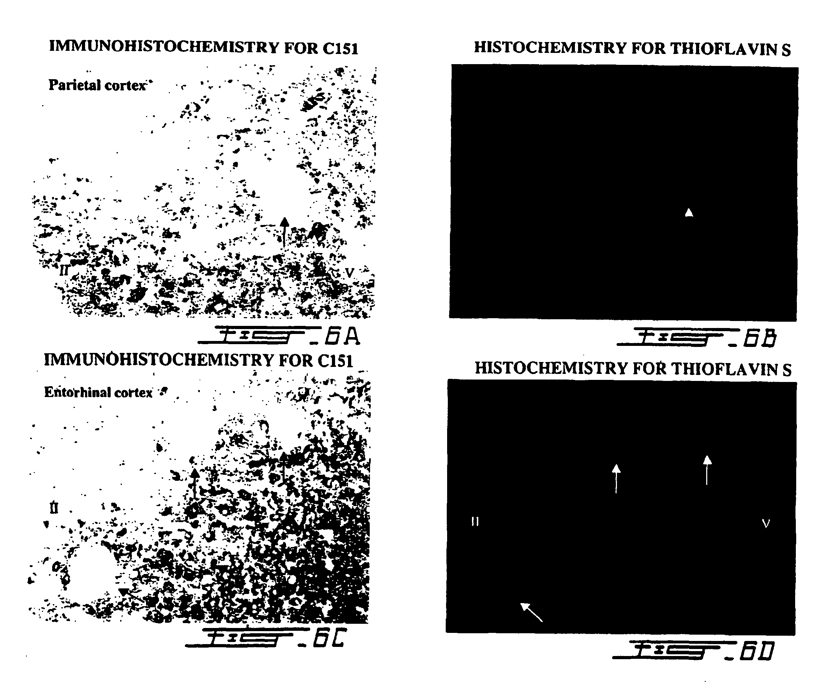

Brain sections were stained with antibodies raised against KLVFFA peptide (D and L forms). As seen in FIGs 5A to 5D and 6A to 6D, the antibodies were not capable of binding to aggregated (ThioS positive) Aβ. It can be seen from both sets of figures, which were stained for both plaques (using ThioS) and Aβ peptide (using the antibodies raised) that the antibodies recognize Aβ at the surface of the cells but are not capable of binding to plaques. These results show that the anti-KLVFFA peptide antibody does not bind to aggregated Aβ. These results clearly prove that the antibody recognizes only the non-aggregated form and blocks fibrillogenesis. By having such activity, the vaccine of the present invention 1) prevents Aβ from organizing itself into a fibril and 2) prevents an inflammatory response being triggered by such an antibody binding to an insoluble form, since the antibody is not able to bind to aggregated Aβ. Furthermore, by maintaining Aβ in a soluble form, such antibody may favor the clearance of Aβ from the brain prior to its organization as a fibril. Such antibody would also maintain a beneficial equilibrium between the concentration of Aβ in the brain vs. the plasma.

### EXAMPLE IV

### Effect of Antibodies Against D- and L- Ap (13-21-KLH) Peptide Vaccine on Amyloid-β Accumulation in the Brain and Plasma

Adult TgCRND8 mice (which carry a transgene expressing the human Amyloid Precursor Protein (hAPP)) were immunized with D or L peptide at 9 weeks of age, and every two weeks thereafter (a total of 5 injections). Animals were sacrificed 10-14 days after the last boost and the accumulation of amyloid-β in the brain and plasma of the mice was determined. Brains were homogenized as follows: First, brains were frozen and the frozen tissue was weighed. Tissue was then homogenized in 4 volumes of 50 mM Tris-HCl pH 8.0 containing 1x protease inhibitor cocktail (Calbiochem #539131) using a Wheaton Overhead Stirrer, speed 3.6. Samples were vortexed and spun in an Eppendorf centrifuge at 16,000x g for 20 min at 4°C. 150 µL, of the supernatant was added to 250 µL 8 M guanidium/50 mM Tris-HCl pH8.0. The mixture was vortexed and stored at -80°C. The pellet was thawed and resuspended by vortexing in 7 volumes of 5 M guanidium/50 mM Tris pH8.0 and stored at -80°C. The supernatant and pellet were thawed and sonicated for 15 min at 80°C in an Elma Ultrasonic bath, then frozen on dry ice for 15 min, then thawed and sonicated in an Elma Ultrasonic bath at 80°C for 10 min. Samples were then spun at 16,000x g for 20 min at 4°C, and the supernatant was kept for amyloid-β quantification. Amyloid-β40 and amyloid-β42 were quantified using a human β Amyloid ELISA kit from Biosource International (catalog #88-344).

Specific plasma antibody titers were determined using a standard ELISA procedure (shown in Table 2, *supra*). The data in table 2 indicates that immunization with the [D] 13-21 peptide was much more effective at inducing an immune response than immunization with the [L] 13-21 peptide.

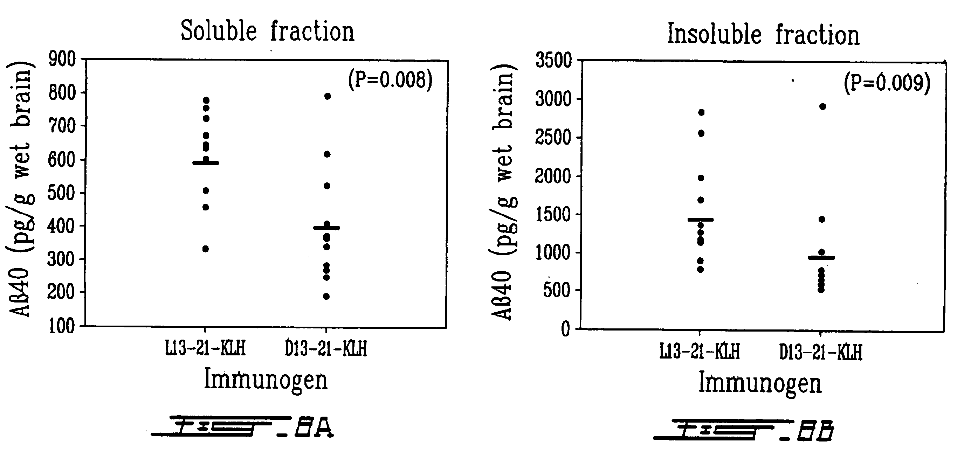

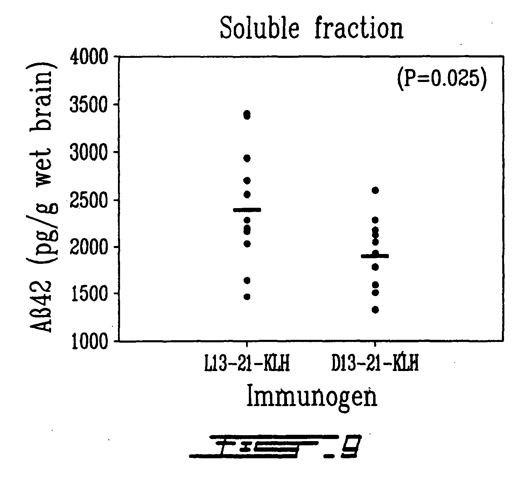

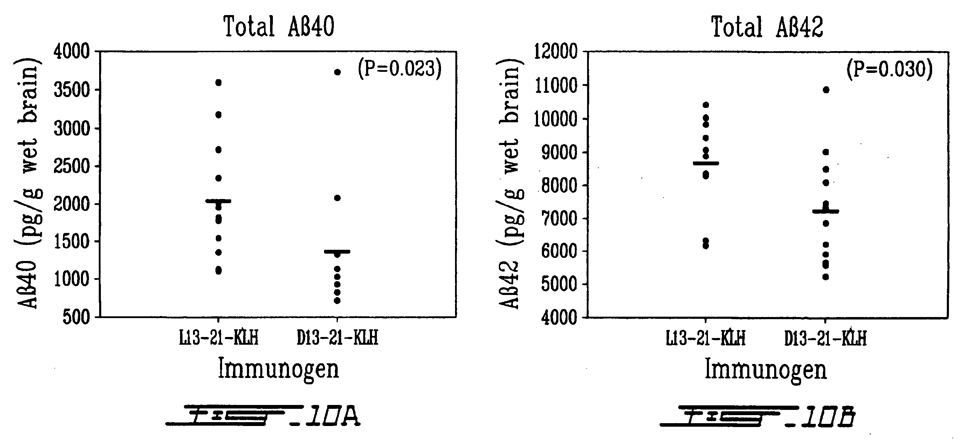

The results also showed a reduction in both soluble and fibrillar (insoluble) Aβ40 level (FIGs. 8A and 8B), a reduction in both soluble and fibrillar Aβ42 level (FIG. 9) and a reduction in total Aβ level (FIGs. 10A and 10B) in the brain of TgCRND8 mice after immunization with [D]13-21-KLH. The results indicate that immunization with D peptides was effective at reducing Aβ levels. Also, D peptides were much more potent than L peptides, as only immunization with D peptides reduced Aβ levels, and immunization with L peptides did not reduce Aβ levels.

### EXAMPLE V

### Passive Immunization Against Amyloid-beta

Humanized antibodies are raised and obtained from transgenic animals such as Xenomouse® (Abgenix, Freemont, CA), by exposing the animal to an antigenic amount of an all-D Amyloid-β (10-22). An antigenic amount ranges from 10ng to 10mg of the all-D Amyloid-β peptide. The immune response can be enhanced -by the use of adjuvants as described, thus permitting lower concentrations of the immunogen to be used. The serum antibody levels are monitored through ELISA or other methods of titering an immune response. The antibodies may be directly recovered from the serum of the animal, or B-cells expressing such antibodies may be recovered from an immunized animal for creation of hybridoma lines. The humanized antibodies are purified from serum, ascites or cell culture medium by Protein G chromatography or similar methods of antibody purification.

The antibodies, raised directly in the second or donor mammal or indirectly by creation of a hybridoma line derived from an immune cell from the donor mammal, can be used to immunize a first or subject mammal suffering from an amyloid related disease or disorder, or to prevent an amyloid related disease or disorder. The first mammal is provided with a therapeutically or prophylactically effective dose of the anti-Aβ antibodies. Antibody fragments or primed immune cells such as B-cells, T-cells, or primed antigen presenting cells can also be obtained from a donor, preferably a human donor and more preferably a syngeneic human donor. A therapeutically effective dose is identified as one which decreases the formation or mass of fibrillar plaques in the first mammal, by at least 5 to 50% or greater, as measured by the techniques and assays described. A 100% or greater reduction in plaque formation or mass is considered a therapeutic endpoint. Physicians or other medical professionals can monitor dosing regimens to achieve a therapeutic effect or endpoint.

## Claims

1. Use of a peptide for the manufacture of a medicament for preventing or treating an amyloid-related disease by inducing an immune response against amyloid-β in a mammal, wherein said peptide consists of an amino acid sequence as set forth in SEQ ID NO:8, or an amino acid sequence as set forth in SEQ ID NO:9, and wherein amino acids of said SEQ ID NO:8 or SEQ ID NO:9 consist entirely of [D]-amino acids.

2. The use according to claim 1, wherein said peptide further comprises:
(a) an N-terminal substituent selected from the group consisting of: hydrogen, lower alkyl group that is either acyclic or cyclic and has 1 to 8 carbon atoms, aromatic group, heterocyclic group, and acyl group; and
(b) a C-terminal substituent selected from the group consisting of hydroxy, alkoxy, aryloxy, unsubstituted and substituted amino groups.

3. The use according to any one of claims 1 and 2, comprising administering an antigenic amount of said peptide to a first mammal wherein said peptide induces an immune response against amyloid-β in said first mammal, recovering antibodies or immune cells from said first mammal, and providing a therapeutically or prophylactically effective dose of said antibodies or immune cells to a second mammal.

4. The use according to any one of claims 1 to 3, wherein said peptide is conjugated to a carrier.

5. The use according to claim 4, wherein said carrier is selected from the group consisting of: keyhole limpet hemocyanin (KLH), C3d, polysaccharide, bovine serum albumin (BSA), tetanus toxoid, heat shock protein (HSP), ovalbumin and cholera toxin.

6. The use according to any one of claims 1 to 5, wherein said medicament is a vaccine.

7. The use according to 6, wherein said vaccine further comprises an adjuvant.

8. The use according to any one of claims 1 to 5, wherein said medicament is a vaccine composition comprising said peptide and a pharmaceutically acceptable excipient.

9. The use according to claim 8, wherein said pharmaceutically acceptable excipient comprises an adjuvant.

10. The use according to claims 7 and 9, wherein said adjuvant is selected from the group consisting of granulocyte-macrophage colony-stimulating factor, interleukin-12, GM-CSF, synthetic muramyl dipeptide analog, monophosphoryl lipid A, lactic acid bacteria, Al(OH)₃, muramyl dipeptides, saponins, CpG motifs, Freund's muramyl dipeptide, LPS derivatives, heat shock protein (HSP), lipid A derivative, polysaccharides, cholera toxin, killed Bordetella pertussis, lymphotoxin *E. coli,* aluminum salt, alum, mineral oil, iscoms, liposomes, virosomes, archaeosomes, transfersomes, niosomes, cochleates, proteosomes, calcium phosphate, DDA (dimethyldioctadecylammonium bromide), cytokines, hormones and C3d.

11. The use according to any one of claims 1 to 10, wherein said immune response alters levels of soluble amyloid-β in the brain of said mammal.

12. The use according to claim 11, wherein altering brain levels of soluble amyloid-β in said mammal thereby reduces or inhibits fibril formation in said brain.

13. The use according to any one of claims 1 to 12, wherein said medicament is for preventing or treating a neurodegenerative disorder.

14. The use according to claim 13, wherein said neurodegenerative disorder is Alzheimer's disease or cerebral amyloid angiopathy.

## Patentansprüche

1. Verwendung eines Peptids für die Herstellung eines Arzneimittels zur Prävention oder Behandlung einer Amyloid-bezogenen Erkrankung durch Induktion einer Immunantwort gegen Amyloid-*β* in einem Säuger, worin das Peptid aus einer Aminosäuresequenz, wie in SEQ ID NO:8 dargelegt, oder einer Aminosäuresequenz, wie in SEQ ID NO:9 dargelegt, besteht, und worin die Aminosäuren von SEQ ID NO:8 oder SEQ ID NO:9 vollkommen aus [D]-Aminosäuren bestehen.

2. Verwendung nach Anspruch 1, worin das Peptid weiter Folgendes umfasst:
(a) einen N-terminalen Substituenten, der aus der Gruppe ausgewählt ist, bestehend aus: Wasserstoff, einer niederen Alkylgruppe, die entweder acyclisch oder cyclisch ist und 1 bis 8 Kohlenstoffatom(e), eine aromatische Gruppe, heterocyclische Gruppe und Acylgruppe aufweist; und
(b) einen C-terminalen Substituenten der aus der Gruppe ausgewählt ist, die aus Hydroxy-, Alkoxy-, Aryloxy-, unsubstituierten und substituierten Aminogruppen besteht.

3. Verwendung nach einem der Ansprüche 1 und 2, umfassend die Verabreichung einer antigenisch Menge des Peptids an einen ersten Säuger, worin das Peptid eine Immunantwort gegen Amyloid-*β* im ersten Säuger induziert, Rückgewinnung von Antikörpern oder Immunzellen aus dem ersten Säuger und Bereitstellung einer therapeutisch oder prophylaktisch wirksamen Dosis von Antikörpern oder Immunzellen an einen zweiten Säuger.

4. Verwendung nach einem der Ansprüche 1 bis 3, worin das Peptid an einen Träger konjugiert ist.

5. Verwendung nach Anspruch 4, worin der Träger aus der Gruppe ausgewählt ist, bestehend aus: Keyhole Limpet-Hämocyanin (KLH), C3d, Polysaccharid, bovinem Serumalbumin (BSA), Tetanus-Toxoid, Hitzeschockprotein (HSP), Ovalbumin und Choleratoxin.

6. Verwendung nach einem der Ansprüche 1 bis 5, worin das Arzneimittel ein Vakzin darstellt.

7. Verwendung nach Anspruch 6, worin das Vakzin weiter ein Adjuvanz umfasst.

8. Verwendung nach einem der Ansprüche 1 bis 5, worin das Arzneimittel eine Vakzin-Zusammensetzung darstellt, umfassend das Peptid und einen pharmazeutisch verträglichen Hilfsstoff.

9. Verwendung nach Anspruch 8, worin der pharmazeutisch verträgliche Hilfsstoff ein Adjuvanz umfasst.

10. Verwendung nach den Ansprüchen 7 und 9, worin das Adjuvanz aus der Gruppe ausgewählt ist, bestehend aus: Granulocyten-Makrophagen-koloniestimulierendem Faktor (GM-CSF), Interleukin-12, einem synthetischem Muramyl-Dipeptid-Analogon, Monophosphoryl-Lipid A, Milchsäurebakterien, Al(OH)₃, Muramyl-Dipeptiden, Saponinen, CpG-Motiven, Muramyl-Dipeptid nach Freund, LPS-Derivaten, Hitzeschockprotein (HSP), einem Lipid-A-Derivat, Polysacchariden, Choleratoxin, abgetöteten Bordetella pertussis, *E. coli*-Lymphotoxin, Aluminiumsalz, Alaun, Mineralöl, immunstimulierenden Komplexen (ISCOMS), Liposomen, Virosomen, Archaeosomen, Transfersomen, Niosomen, Cochleaten, Proteosomen, Calciumphosphat, DDA (Dimethyldioctadecylammoniumbromid), Cytokinen, Hormonen und C3d.

11. Verwendung nach einem der Ansprüche 1 bis 10, worin die Immunantwort die Spiegel von löslichem Amyloid-β im Gehirn des Säugers verändert.

12. Verwendung nach Anspruch 11, worin die Veränderung der Spiegel von löslichem Amyloid-*β* im Gehirn des Säugers folglich die Fibrillenbildung im Gehirn reduziert oder inhibiert.

13. Verwendung nach einem der Ansprüche 1 bis 12, worin das Arzneimittel zur Prävention oder Behandlung einer neurodegenerativen Erkrankung vorgesehen ist.

14. Verwendung nach Anspruch 13, worin die neurodegenerative Erkrankung die Alzheimer-Krankheit oder cerebrale Amyloid-Angiopathie darstellt.

## Revendications

1. Utilisation d'un peptide dans la fabrication d'un médicament indiqué dans la prévention ou le traitement d'une maladie liée à l'amyloïde par l'induction d'une réponse immunitaire anti-amyloïde *β* chez un mammifère, où ledit peptide consiste en une séquence d'acides aminés telle que définie à la SÉQ ID N° : 8 ou en une séquence d'acides aminés telle que définie à la SÉQ ID N° : 9, et où les acides aminés desdites séquences SÉQ ID N° : 8 ou SÉQ ID N° : 9 sont entièrement composées d'acides aminés en configuration D.

2. Utilisation selon la revendication 1, où ledit peptide comprend également :
(a) un substituant en position N-terminale sélectionné dans le groupe consistant en un atome d'hydrogène, un groupement alkyle inférieur qui est soit acyclique, soit cyclique et comporte de 1 à 8 atomes de carbone, un groupement aromatique, un groupement hétérocyclique et un groupement acyle; et
(b) un substituant en position C-terminale sélectionné dans le groupe consistant en un groupement hydroxy, alkoxy, aryloxy et un groupement amino non substitué et substitué.

3. Utilisation selon l'une quelconque des revendications 1 et 2, qui comprend l'administration dudit peptide en une quantité antigénique à un premier mammifère chez qui ledit peptide induit une réponse immunitaire anti-amyloïde *β*, la récupération des anticorps ou cellules immunitaires dudit premier mammifère et l'administration desdits anticorps ou cellules immunitaires à un second mammifère à une dose efficace sur le plan thérapeutique ou prophylactique.

4. Utilisation selon l'une quelconque des revendications 1 et 3, où ledit peptide est conjugué à un vecteur.

5. Utilisation selon la revendication 4, où ledit vecteur est sélectionné dans le groupe consistant en l'hémocyanine de patelle (KLH), le fragment C3d, un polysaccharide, l'albumine sérique bovine (ASB), l'anatoxine tétanique, une protéine de choc thermique (PCT), l'ovalbumine et la toxine cholérique.

6. Utilisation selon l'une quelconque des revendications 1 à 5, où ledit médicament est un vaccin.

7. Utilisation selon la revendication 6, où ledit vaccin comprend également un adjuvant.

8. Utilisation selon l'une quelconque des revendications 1 à 5, où ledit médicament est une composition vaccinale comprenant ledit peptide et un excipient pharmaceutiquement acceptable.

9. Utilisation selon la revendication 8, où ledit excipient pharmaceutiquement acceptable comprend un adjuvant.

10. Utilisation selon les revendications 7 et 9, où ledit adjuvant est sélectionné dans le groupe consistant en le facteur de stimulation des colonies de granulocytes-macrophages (GM-CSF), l'interleukine-12, un analogue de synthèse du muramyl-dipeptide (MDP), le monophosphoryle-lipide A (MPL), des bactéries à acide lactique, Al(OH)₃, des muramyl-dipeptides, des saponines, des motifs CpG, l'adjuvant de Freund avec muramyl-dipeptide, des dérivés de lipopolysaccharides (LPS), une protéine de choc thermique (PCT), un dérivé du lipide A, des polysaccharides, la toxine cholérique, *Bordetella pertussis* tué, la lymphotoxine d'*E. coli,* un sel d'aluminium, l'alun, une huile minérale, des iscomes, des liposomes, des virosomes, des archaeosomes, des transfersomes, des niosomes, des cochléates, des protéosomes, le phosphate de calcium, le DDA (bromure de diméthyldioctadécylammonium), des cytokines, des hormones et le fragment C3d.

11. Utilisation selon l'une quelconque des revendications 1 à 10, où ladite réponse immunitaire modifie les taux d'amyloïde *β* soluble dans le cerveau dudit mammifère.

12. Utilisation selon la revendication 11, où la modification des taux cérébraux d'amyloïde *β* soluble chez ledit mammifère réduit ou inhibe la formation de fibrilles dans ledit cerveau.

13. Utilisation selon l'une quelconque des revendications 1 à 12, où ledit médicament est indiqué dans la prévention ou le traitement d'une maladie neurodégénérative.

14. Utilisation selon la revendication 13, où ladite maladie neurodégénérative est la maladie d'Alzheimer ou l'angiopathie amyloïde cérébrale.
